# EUROPEAN PATENT SPECIFICATION

(11) **EP 0 925 298 B1**
(45) Date of publication and mention of the grant of the patent: **14.04.2004**
(21) Application number: 97929464.2
(22) Date of filing: 21.07.1997
(51) Int. Cl.: C07D 471/04, A61K 31/505, C07D 475/02, A61K 31/495, C07D 498/04, C07D 215/22, C07D 405/12, A61K 31/47

(54) **SUBSTITUTED 6,6-HETERO-BICYCLIC DERIVATIVES**
SUBSTITUIERTE 6,6-HETEROBICYCLISCHE DERIVATE
DERIVES 6,6-HETERO-BICYCLIQUES SUBSTITUES

(30) Priority: 27.08.1996 US 24659 P
(43) Date of publication of application: 30.06.1999
(73) Proprietor: PFIZER INC., New York, N.Y. 10017 (US)
(72) Inventor: CHEN, Yuhpyng, Liang, Waterford, CT 06385 (US)
(74) Representative: Atkinson, Jonathan David Mark
(86) International application number: PCT/IB1997/000904
(87) International publication number: WO 1998/008846

(56) References cited:
- EP-A- 0 729 758
- EP-A- 0 773 023
- EP-A- 0 778 277
- WO-A-94/13676
- WO-A-94/13677
- WO-A-95/33750
- WO-A-95/34563
- WO-A-96/35689

## Description

### Background of the invention

This invention relates to certain pharmaceutically active substituted 6,6-hetero-bicyclic derivatives, pharmaceutical compositions containing them and methods of administering them to subjects in need of their corticotropin releasing factor antagonist activity.

The substituted heterocyclic derivatives claimed in this case exhibit activity as corticotropin releasing factor (hormone) CRF (CRH) antagonists.

CRF antagonists are mentioned in U.S. Patents 4,605,642 and 5,063,245 referring to peptides and pyrazolinones, respectively. They are also referred to in the following: PCT Patent Application PCT/IB95/00439 (WO95/33750), which designates the United States and was filed on June 6, 1995 and published on December 14, 1995; PCT Patent Application PCT/IB95/00373 (WO95/34563), which designates the United States and was filed on May 18, 1995 and published on December 21, 1995; U.S. Patent Application 08/448,539 (WO94/13676), which was filed in the PCT on Nov. 12, 1993 and entered the U.S. national phase on June 14, 1995; PCT Patent Application WO 95/10506, which was filed on October 12, 1993 and published on April 20, 1995, and U.S. Patent Application 08/481,413 (WO94/13677), which was filed in the PCT on November 26, 1993 and entered the U.S. national phase on July 24, 1995; U.S. Patent Application 08/254,820 (WO94/13661), which was filed on April 19, 1995; Provisional U.S. Patent Application 60/008,396 (EP0778277), which was filed on December 8, 1995; and Provisional U.S. Patent Application 60/006,333 (EP0773023), which was filed on November 8, 1995. All the foregoing patent applications are incorporated herein by reference in their entireties.

The importance of CRF antagonists is set out in the literature, e.g., P. Black, Scientific American SCIENCE & MEDICINE, 1995, p. 16-25; T. Lovenberg, et al., Current Pharmaceutical Design, 1995, 1, 305-316; and United States Patent 5,063,245, which is referred to above. A recent outline of the different activities possessed by CRF antagonists is found in M. J. Owens et al., Pharm. Rev., Vol. 43, pages 425 to 473 (1991), also incorporated herein by reference. Based on the research described in these two and other references, CRF antagonists are effective in the treatment of a wide range of stress-related illnesses, mood disorders such as depression, major depressive disorder, single episode depression, recurrent depression, child abuse induced depression, postpartum depression, dysthemia, bipolar disorders and cyclothymia; chronic fatigue syndrome; eating disorders such as anorexia and bulimia nervosa; generalized anxiety disorder; panic disorder; phobias; obsessive-compulsive disorder, post-traumatic stress disorder, pain perception such as fibromyalgia; headache; gastrointestinal diseases; hemorrhagic stress; ulcers; stress-induced psychotic episodes; fever; diarrhea; post-operative ileus, colonic hypersensitivity; irritable bowel syndrome; Crohn's disease; spastic colon; inflammatory disorders such as rheumatoid arthritis and osteoarthritis; pain; asthma; psoriasis; allergies; osteoporosis; premature birth; hypertension, congestive heart failure; sleep disorders; neurodegenerative diseases such as Alzheimer's disease, senile dementia of the Alzheimer's type, multiinfarct dementia, Parkinson's disease, and Huntington's disease; head trauma; ischemic neuronal damage; excitotoxic neuronal damage; epilepsy; stroke; spinal cord trauma; psychosocial dwarfism; euthyroid sick syndrome; syndrome of inappropriate antidiarrhetic hormone; obesity; chemical dependencies and addictions; drug and alcohol withdrawal symptoms; infertility, cancer; infertility; muscular spasms; urinary incontinence; hypoglycemia and immune dysfunctions including stress induced immune dysfunctions, immune suppression and human immunodeficiency virus infections; and stress-induced infections in humans and animals.

The compounds of this invention are also believed to be inhibitors of CRH binding protein and therefore useful in the treatment of disorders the treatment of which can be effected or facilitated by inhibiting such protein. Examples of such disorders are Alheimer's disease and obesity.

### Summary of the Invention

The present invention relates to compounds of the formula 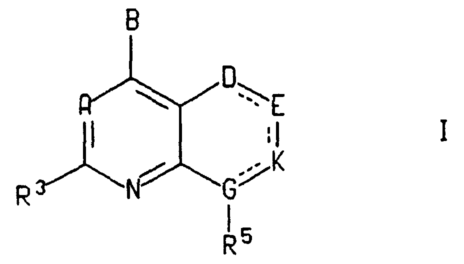
the dashed lines represent optional double bonds;
A is nitrogen or CR⁷;
B is -NR¹R², -CR¹R²R¹⁰, -C(=CR²R¹¹)R¹, -NHCR¹R²R¹⁰, -OCR¹R²R¹⁰, -SCR¹R²R¹⁰, -CR²R¹⁰NHR¹, -CR²R¹⁰OR¹, -CR²R¹⁰SR¹ or -COR²;
G is nitrogen or CR⁴ and is single bonded to all atoms to which it is attached, or G is carbon and is double bonded to K;
K is nitrogen or CR⁶ when double bonded to G or E, or K is oxygen, sulfur, C=O, C=S, CR⁶R¹² or NR⁸ when single bonded to both adjacent ring atoms, or K is a two atom spacer, wherein one of the two ring atoms of the spacer is oxygen, nitrogen, sulfur, C=O, C=S, CR⁶R¹², NR⁶ or CR⁶, and the other is CR⁶R¹² or CR⁹;
D and E are each, independently, C=O, C=S, sulfur, oxygen, CR⁴R⁶ or NR⁸ when single bonded to both adjacent ring atoms, or nitrogen or CR⁴ when it is double bonded to an adjacent ring atom;
the 6- or 7-membered ring that contains D, E, K and G may contain from one to three double bonds, from zero to two heteroatoms selected from oxygen, nitrogen and sulfur, and from zero to two C=O or C=S groups, wherein the carbon atoms of such groups are part of the ring and the oxygen and sulfur atoms are substituents on the ring;
R¹ is C₁-C₆ alkyl optionally substituted with from one or two substituents independently selected from hydroxy, fluoro, chloro, bromo, iodo, C₁-C₄ alkoxy, CF₃, -C(=O)(C₁-C₄alkyl), -C(=O)-O-(C₁-C₄)alkyl, -OC(=O)(C₁-C₄ alkyl), -OC(=O)N(C₁-C₄ alkyl)(C₁-C₂ alkyl), -NHCO(C₁-C₄ alkyl), -COOH, -COO(C₁-C₄ alkyl), -CONH(C₁-C₄ alkyl), - CON(C₁-C₄ alkyl)(C₁-C₂ alkyl), -S(C₁-C₄ alkyl), -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl), wherein each of the C₁-C₄ alkyl groups in the foregoing R¹ groups may optionally contain one or two double or triple bonds;
R² is C₁-C₁₂ alkyl which may optionally contain from one to three double or triple bonds, aryl or (C₁-C₄ alkylene)aryl, wherein said aryl and the aryl moiety of said (C₁-C₄ alkylene)aryl is selected from phenyl, naphthyl, thienyl, benzothienyl, pyridyl, quinolyl, pyrazinyl, pyrimidinyl, imidazolyl, furanyl, benzofuranyl, benzothiazolyl, isothiazolyl, pyrazolyl, pyrrolyl, indolyl, pyrrolopyridyl, oxazolyl and benzoxazolyl; C₃-C₈ cycloalkyl or (C₁-C₆ alkylene)(C₃-C₈ cycloalkyl), wherein one or two of the carbon atoms of said cycloalkyl and the 5 to 8 membered cycloalkyl moieties of said (C₁-C₆ alkylene)(C₃-C₈ cycloalkyl may optionally and independently be replaced by an oxygen or sulfur atom or by NZ wherein Z is hydrogen, C₁-C₄ alkyl or benzyl, and wherein each of the foregoing R² groups may optionally be substituted with from one to three substituents independently selected from chloro, fluoro, hydroxy and C₁-C₄ alkyl, or with one substituent selected from C₁-C₆ alkoxy, -OC(=O)(C₁-C₆ alkyl), -OC(=O)N(C₁-C₄ alkyl)(C₁-C₂ alkyl), -S(C₁-C₆ alkyl), amino, -NH(C₁-C₂ alkyl), -N(C₁-C₂ alkyl)(C₁-C₄ alkyl), - N(C₁-C₄ alkyl)-CO-(C₁-C₄ alkyl), -NHCO(C₁-C₄ alkyl), -COOH, -COO(C₁-C₄ alkyl), -CONH(C₁-C₄ alkyl), -CON(C₁-C₄ alkyl)(C₁-C₂ alkyl), -SH, -CN, -NO₂, -SO(C₁-C₄ alkyl), -SO₂(C₁-C₄ alkyl), -SO₂NH(C₁-C₄ alkyl) and -SO₂N(C₁-C₄ alkyl)(C₁-C₂ alkyl);
-NR¹R² or CR¹R²R¹⁰ may form a ring selected from saturated 3 to 8 membered rings, the 5 to 8 membered rings of which may optionally contain one or two double bonds, and wherein one or two of the ring carbon atoms of such 5 to 8 membered rings may optionally and independently be replaced by an oxygen or sulfur atom or by NZ² wherein Z² is hydrogen, benzyl or C₁-C₄ alkyl;
R³ is hydrogen, C₁-C₄ alkyl, -O(C₁-C₄ alkyl), chloro, fluoro, bromo, iodo, -S(C₁-C₄ alkyl) or -SO₂(C₁-C₄ alkyl);
each R⁸, R⁹ and R¹² is selected, independently, from hydrogen and C₁-C₂ alkyl;
each R⁴ and R⁶ that is attached to a carbon atom is selected, independently, from hydrogen, C₁-C₆ alkyl, fluoro, chloro, bromo, iodo, hydroxy, hydroxy (C₁-C₂ alkyl), trifluoromethyl, cyano, amino, nitro, -O(C₁-C₄ alkyl), -N(C₁-C₄ alkyl)(C₁-C₂ alkyl), -CH₂SCH₃, -S(C₁-C₄ alkyl), -CO(C₁-C₄ alkyl), -C(=O)H or -C(=O)O(C₁-C₄ alkyl), wherein each of the C₁-C₂ alkyl moieties in the foregoing R⁴ and R⁶ groups may optionally contain one double or triple bond; and R⁶, when attached to a nitrogen atom, is selected from hydrogen and (C₁-C₄)alkyl;
R⁵ is substituted phenyl, naphthyl, pyridyl or pyrimidyl, wherein each of the foregoing R⁵ groups is substituted with from two to four substituents R¹³, wherein up to three of said substituents may be selected, independently, from chloro, C₁-C₆ alkyl, - O(C₁-C₆ alkyl) and -(C₁-C₆ alkylene)O(C₁-C₆ alkyl), and wherein one of said substituents may be selected, independently, from bromo, iodo, formyl, cyano, trifluoromethyl, nitro, amino, -NH(C₁-C₄ alkyl), -N(C₁-C₂ alkyl)(C₁-C₆ alkyl), - C(=O)O(C₁-C₄ alkyl), -C(=O)(C₁-C₄ alkyl), -COOH, -SO₂NH(C₁-C₄ alkyl), -SO₂N(C₁-C₂ alkyl)(C₁-C₄ alkyl), -SO₂NH₂, -NHSO₂(C₁-C₄ alkyl), -(C₀-C₁alkylene)-S-(C₁-C₂alkyl), -(C₀-C₁alkylene)-SO-(C₁-C₂alkyl), -(C₀-C₁ alkylene)-SO₂-(C₁-C₂alkyl) and (C₁-C₄alkylene)-OH, and wherein each of the C₁-C₄ alkyl and C₁-C₆ alkyl moieties in the foregoing R⁵ groups may optionally be substituted with one or two substituents independently selected from fluoro, hydroxy, amino, methylamino, dimethylamino and acetyl;
R⁷ is hydrogen, methyl, halo (e.g., chloro, fluoro, iodo or bromo), hydroxy, methoxy, -C(=O)(C₁-C₂ alkyl), -C(=O)O(C₁-C₂ alkyl), hydroxymethyl, trifluoromethyl or formyl;
R¹⁰ is hydrogen, hydroxy, methoxy or fluoro; and
R¹¹ is hydrogen or C₁-C₄ alkyl;
with the proviso that in the ring containing D, E, K and G of formula I, there can not be two double bonds adjacent to each other;
and the pharmaceutically acceptable salts of such compounds.

Examples of more specific embodiments of formula I are the following, wherein X is oxygen, sulfur or NR⁸, wherein R⁸ is defined as above, each dashed line represents an optional double bond and (R)ₙ represents from zero to four substituents, wherein such substitutents are as defined above in the definition of formula I.

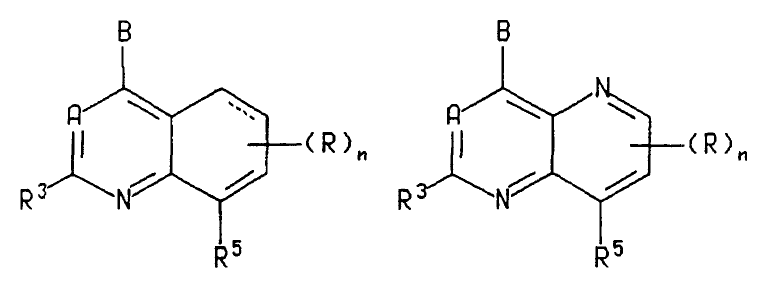

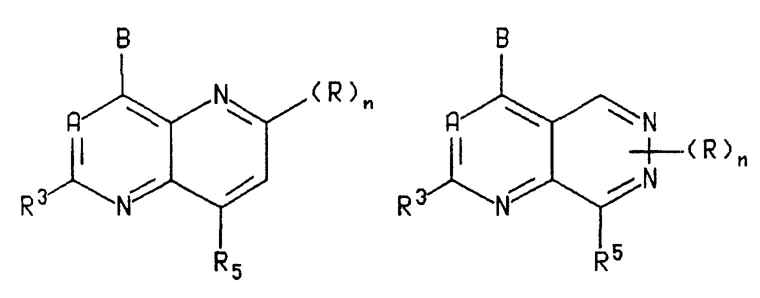

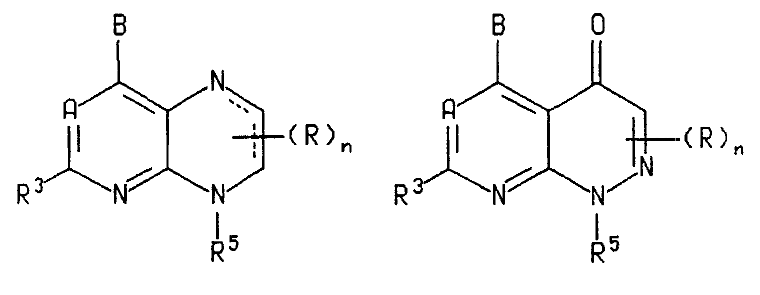

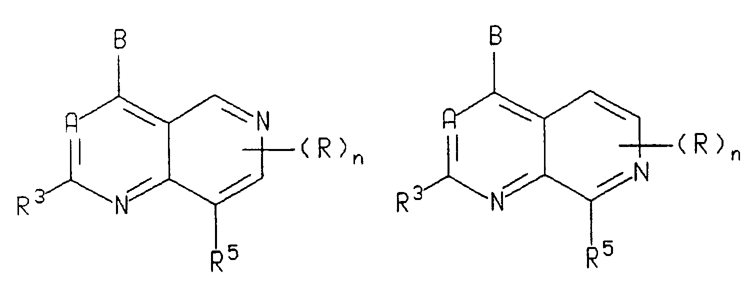

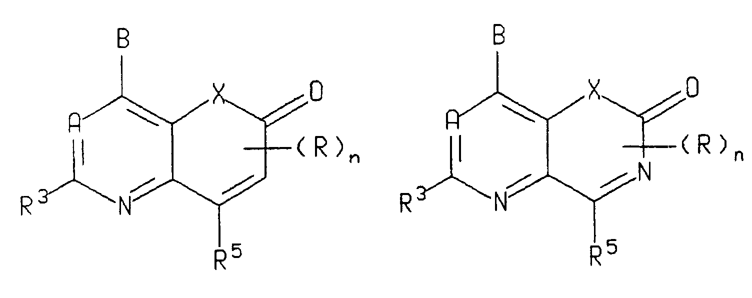

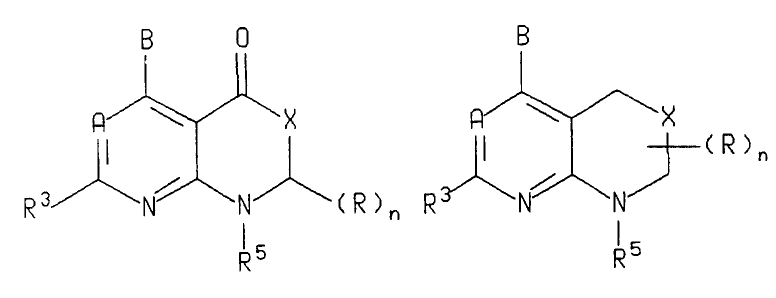

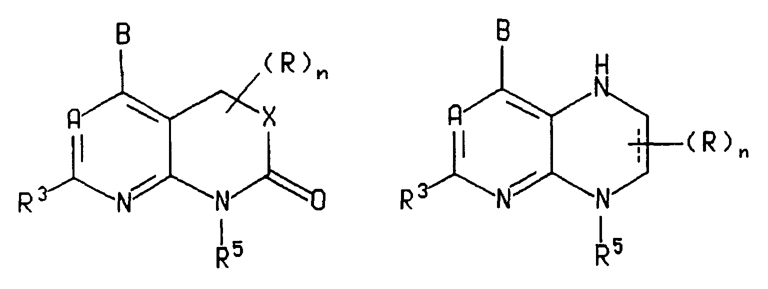

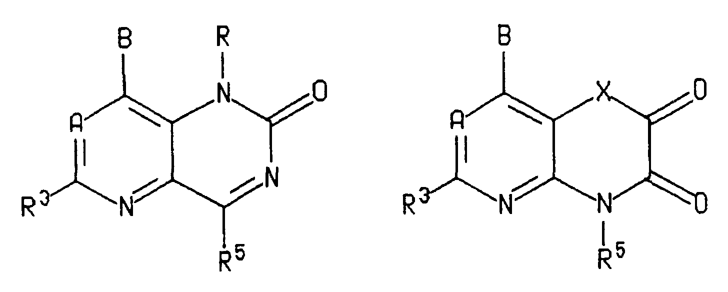

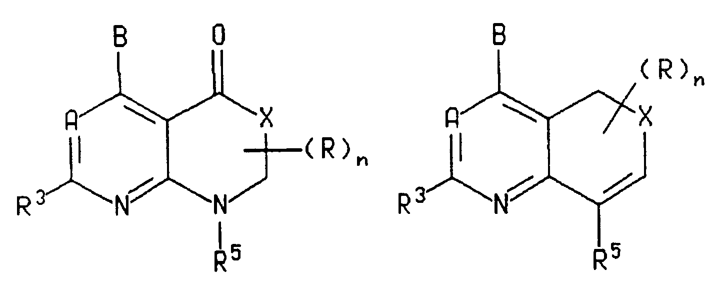

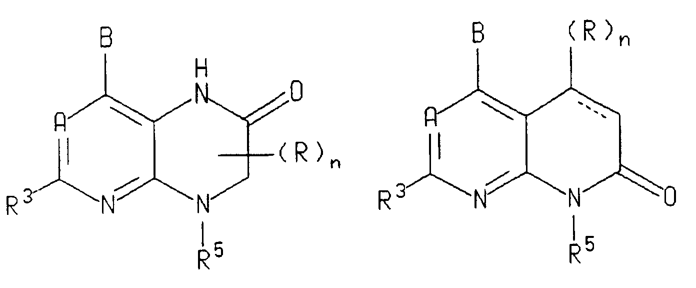

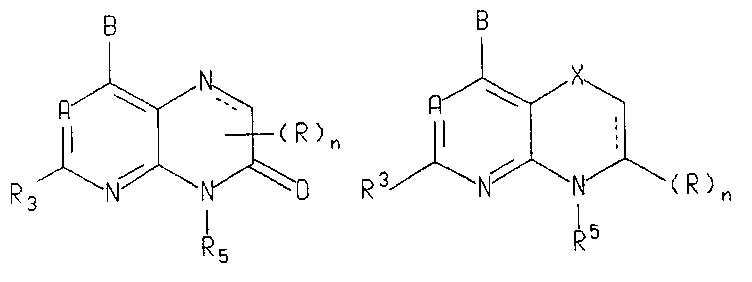

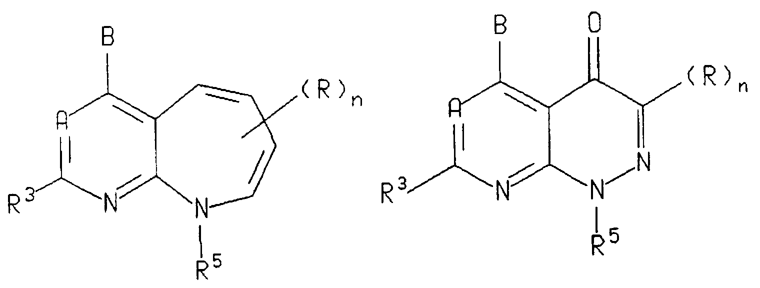

More specific embodiments of the invention include compounds of the formula I wherein B is -CHR¹R², -NR¹R², -NHCHR¹R², -OCHR¹R², or -SCHR¹R², and R¹ is C₁-C₆ alkyl, which may optionally be substituted with one hydroxy, fluoro, CF₃ or C₁-C₄ alkoxy group and may optionally contain one double or triple bond; and R² is benzyl or C₁-C₆ alkyl, which may optionally contain one double or triple bond, wherein said C₁-C₆ alkyl and the phenyl moiety of said benzyl may optionally be substituted with one fluoro, hydroxy, CF₃, C₁-C₂ alkyl, C₁-C₂ alkoxy or chloro group.

Preferably G is carbon and the ring containing D, E, K and G is a benzo ring. More preferably R³ is methyl.

In an embodiment R⁵ is di- or tri-substituted phenyl at the ortho or para positions in which the two or three substituents are independently selected from C₁-C₄ alkyl, cyclopropyl, -O-(C₁-C₄ alkyl), -(C₁-C₄ alkylene)-O-(C₁-C₄ alkyl), CF₃, OCF₃, CHO, -(C₁-C₄ alkylene)-OH, chloro, fluoro, bromo and iodo, wherein each of the foregoing C₁-C₄ alkyl groups may optionally contain one double or triple bond.

In an embodiment R⁵ is di- or tri-substituted pyridyl at the ortho or para positions in which the two or three substituents are independently selected from C₁-C₄ alkyl, cyclopropyl, -O-(C₁-C₄ alkyl), -(C₁-C₄ alkylene)-O-(C₁-C₄ alkyl), CF₃, OCF₃, CHO, -(C₁-C₄ alkylene)-OH, chloro, fluoro, bromo and iodo, wherein each of the foregoing C₁-C₄ alkyl groups may optionally contain one double or triple bond.

In an embodiment G is N; D--E--K is C(C_{zero}-C₁alkyl)-O-C(C_{zero}-C₁ alkyl)(C_{zero}-C₁ alkyl) Preferably, G is N; D--E--K is CH₂-O-CH₂.

In an embodiment R³ is methyl.

In an embodiment R⁵ is di- or tri-substituted phenyl at the ortho or para positions in which the two or three substituents are independently selected from C₁-C₄ alkyl, cyclopropyl, -O-(C₁-C₄ alkyl), -(C₁-C₄ alkylene)-O-(C₁-C₄ alkyl), CF₃, OCF₃, CHO, -(C₁-C₄ alkylene)-OH, chloro, fluoro, bromo and iodo, wherein each of the foregoing C₁-C₄ alkyl groups may optionally contain one double or triple bond.

In an embodiment R⁵ is di- or tri-substituted pyridyl at the ortho or para positions in which the two or three substituents are independently selected from C₁-C₄ alkyl, cyclopropyl, -O-(C₁-C₄ alkyl), -(C₁-C₄ alkylene)-O-(C₁-C₄ alkyl), CF₃, OCF₃, CHO, -(C₁-C₄ alkylene)-OH, chloro, fluoro, bromo and iodo, wherein each of the foregoing C₁-C₄ alkyl groups may optionally contain one double or triple bond.

In an embodiment G is N; D--E--K is O-C(C_{zero}-C₁ alkyl)(C_{zero}-C₁ alkyl)- C(C_{zero}-C₁ alkyl)(C_{zero}-C₁ alkyl), S-C(C_{zero}-C₁ alkyl)(C_{zero}-C₁ alkyl)- C(C_{zero}-C₁ alkyl)(C_{zero}-C₁ alkyl) or N(C_{zero}-C₁ alkyl)-C(C_{zero}-C₁ alkyl)(C_{zero}-C₁ alkyl)- C(C_{zero}-C₁ alkyl)(C_{zero}-C₁ alkyl).

In an embodiment G is N; D--E--K is O-CH₂-CH₂, O-CH=CH, S-CH₂-CH_{2,} S-CH=CH.

Preferably, R³ is methyl.

Preferably, R⁵ is di- or tri-substituted phenyl at the ortho or para positions in which the two or three substituents are independently selected from C₁-C₄ alkyl, cyclopropyl , -O-(C₁-C₄ alkyl), -(C₁-C₄ alkylene)-O-(C₁-C₄ alkyl), CF₃, OCF₃, CHO, -(C₁-C₄ alkylene)-OH, chloro, fluoro, bromo and iodo, wherein each of the foregoing C₁-C₄ alkyl groups may optionally contain one double or triple bond.

More preferably, R⁵ is di-substituted pyridyl at the ortho or para positions in which the two or three substituents are independently selected from C₁-C₄ alkyl, cyclopropyl , -O-(C₁-C₄ alkyl), -(C₁-C₄ alkylene)-O-(C₁-C₄ alkyl), CF₃, OCF₃, CHO, -(C₁-C₄ alkylene)-OH, chloro, fluoro, bromo and iodo, wherein each of the foregoing C₁-C₄ alkyl groups may optionally contain one double or triple bond.

In an embodiment G is N; D--E--K is NH-CH₂-CH₂, NMe-CH₂-CH₂-N-R⁵, NH-CH=CH-N-R⁵, or NCH₃-CH=CH-N-R⁵.

Preferably, R³ is methyl.

Preferably, R⁵ is di- or tri-substituted phenyl at the ortho or para positions in which the two or three substituents are independently selected from C₁-C₄ alkyl, cyclopropyl , -O-(C₁-C₄ alkyl), -(C₁-C₄ alkylene)-O-(C₁-C₄ alkyl), CF₃, OCF₃, CHO, -(C₁-C₄ alkylene)-OH, chloro, fluoro, bromo and iodo, wherein each of the foregoing C₁-C₄ alkyl groups may optionally contain one double or triple bond.

More preferably R⁵ is di-substituted pyridyl at the ortho or para positions in which the two or three substituents are independently selected from C₁-C₄ alkyl, cyclopropyl , -O-(C₁-C₄ alkyl), -(C₁-C₄ alkylene)-O-(C₁-C₄ alkyl), CF₃, OCF₃, CHO, -(C₁-C₄ alkylene)-OH, chloro, fluoro, bromo and iodo, wherein each of the foregoing C₁-C₄ alkyl groups may optionally contain one double or triple bond.

In an embodiment G is N; D--E--K is N=C(C_{zero}-C₁ alkyl)-C(=O), N(C_{zero}-C₁ alkyl)-C(=O)-C(C_{zero}-C₁ alkyl), C(=O)-N(C_{zero}-C₁ alkyl)-C(=O), C(C_{I})=N-C(=O), C(C_{zero}-C₁ alkyl)=N-C(=O), CH₂CH₂CH₂, CH₂-CH₂-C(=O), CH₂-N(C_{zero}-C₁ alkyl)-C(=O).

Preferably, R³ is methyl.

More preferably, R⁵ is di- or tri-substituted phenyl at the ortho or para positions in which the two or three substituents are independently selected from C₁-C₄ alkyl, cyclopropyl, -O-(C₁-C₄ alkyl), -(C₁-C₄ alkylene)-O-(C₁-C₄ alkyl), CF₃, OCF₃, CHO, -(C₁-C₄ alkylene)-OH, chloro, fluoro, bromo and iodo, wherein each of the foergoing C₁-C₄ alkyl groups may optionally contain one double or triple bond.

More preferably, R⁵ is di-substituted pyridyl at ortho or para positions in which the two or three substituents are independently selected from C₁-C₄ alkyl, cyclopropyl, -O-(C₁-C₄ alkyl), - (C₁-C₄ alkylene)-O-(C₁-C₄ alkyl), CF₃, OCF₃, CHO, -(C₁-C₄ alkylene)-OH, chloro, fluoro, bromo and iodo, wherein each of the foregoing C₁-C₄ alkyl groups may optionally contain one double or triple bond.

In an embodiment R³ is methyl and each of R⁴, R⁶, R⁸, R⁹ and R¹² is hydrogen.

In an embodiment R⁵ is di- or tri-substituted phenyl in which the two or three substitutents are independently selected from C₁-C₄ alkyl, -O-(C₁-C₄ alkyl), -(C₁-C₄ alkylene)-O-(C₁-C₄ alkyl), CF₃, -OCF₃, -CHO, -(C₁-C₄ alkylene)-OH, cyano, chloro, fluoro, bromo and iodo, wherein each of the forgoing (C₁-C₄) alkyl groups may optionally contain one double or triple bond.

In an embodiment R³ is methyl, ethyl, chloro or methoxy and each R⁴, R⁶, R⁸, R⁹, and R¹² is, independently, hydrogen or methyl.

In an embodiment R⁵ is di- or tri-substituted phenyl, pyridyl, or pyrimidyl in which the two or three substitutents are independently selected from C₁-C₄ alkyl, -O-(C₁-C₄ alkyl), -(C₁-C₄ alkylene)-O-(C₁-C₄ alkyl), CF₃,- OCF₃, -CHO, -(C₁-C₄ alkylene)-OH, cyano, chloro, fluoro, bromo and iodo, wherein each of the forgoing (C₁-C₄) alkyl groups may optionally contain one double or triple bond;

In an embodiment B is -CHR¹R², -NCHR¹R² or -OCHR¹R², and the CHR¹R² group of B is a cyclopentane ring, a tetrahydrofuran ring or a tetrahydrothienyl ring.

Preferred compounds are compounds of the formula 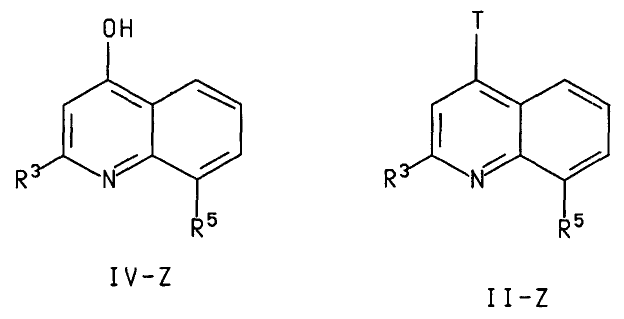 wherein R³, R⁷ and R⁵ are defined as in claim 1 and T is Cl, Br, I or OTf.

Other more specific embodiments of this invention include compounds of the formula I wherein B is or contains an NR¹R² or CR¹R²R¹⁰ moiety which forms a saturated or unsaturated 5-membered carbocyclic ring wherein one of the ring carbon atoms may optionally be replaced by an oxygen or sulfur atom.

Other more specific embodiments of the invention include compounds of formula I wherein R³ is methyl, ethyl, chloro or methoxy; each of R⁴, R⁶, R⁸, R⁹, and R¹² is, independently, hydrogen or methyl; and R⁵ is di- or tri-substituted phenyl, pyridyl, or pyrimidyl, wherein up to three of the substitutents can be selected, independently, from C₁-C₄ alkyl, - O-(C₁-C₄ alkyl) and -(C₁-C₄ alkylene)-O-(C₁-C₄ alkyl), and wherein one of the substituents can be selected, independently, from -(C₀-C₁alkylene)-S-(C₁-C₂alkyl), -(C₀-C₁alkylene)-SO-(C₁-C₂alkyl), -(C₀-C₁alkylene)-SO₂-(C₁-C₂alkyl), CF₃, -OCF₃, -CHO,
-(C₁-C₄ alkylene)-OH, cyano, chloro, fluoro, bromo and iodo, and wherein each of the forgoing (C₁-C₄) alkyl groups may optionally contain one double or triple bond.

Other more specific embodiments of the invention include compounds of the formula I wherein A is N, CH or CCH₃.

Other more specific embodiments of the invention include compounds of the formula I wherein G is N.

Other more specific embodiments of the invention include compounds of the formula I wherein G is carbon and the ring containing D, E, K and G is a benzo ring.

Other more specific embodiments of the invention include compounds of the formula I wherein G is N; D is NH or N(methyl); and E---K is CH₂-CH₂, CH=CH, C(O)-CH₂ or CH₂-C(O).

Other more specific embodiments of the invention include compounds of the formula I wherein G is N and D---E---K is C(O)-O-CH₂, CH₂-O-CH₂, C(O)-CH₂-CH₂, C(O)-CH=CH, CH₂-CH₂-CH₂-, CH₂-CH₂-C(O), CH=CH-C(O), CH=CH-CH₂, CH=CH-NH or CH=CH-NCH₃.

Examples of preferred compounds of the invention are:
4-(butyl-ethyl-amino)-2,6-dimethyl-8-(2,4,6-trimethyl-phenyl)-5,8-dihydro-6H-pyrido [2,3-d]pyrimidin-7-one;
8-(1-ethyl-propoxy)-6-methyl-4-(2,4,6-trimethyl-phenyl)-3,4-dihydro-1H-pyrido[2,3-b] pyrazin-2-one;
8-(1-ethyl-propoxy)-6-methyl-4-(2,4,6-trimethyl-phenyl)-1,2,3,4-tetrahydro-pyrido [2,3-b]pyrazine;
4-(1-ethyl-propoxy)-2-methyl-8-(2,4,6-trimethyl-phenyl)-quinoline;
5-(1-ethyl-propoxy)-7-methyl-1-(2,4,6-trimethyl-phenyl)-1,4-dihydro-2H-3-oxa-1,8-diaza-naphthalene;
5-(1-ethyl-propoxy)-7-methyl-1-(2,4,6-trimethyl-phenyl)-1,2-dihydro-3-oxa-1,8-diaza- naphthalen-4-one;
8-(1-ethyl-propoxy)-1,6-dimethyl-4-(2,4,6-trimethyl-phenyl)-1,2,3,4-tetrahydro-pyrido[2,3-b]pyrazine;
(1-ethyl-propyl)-[2-methyl-8-(2,4,6-trimethyl-phenyl)-quinolin-4-yl]-amine;

Other examples of compounds of the formula I are the following:
4-(butyl-ethyl-amino)-2,6-dimethyl-8-(2,6-dimethyl-4-bromo-phenyl)-5,8-dihydro-6H-pyrido[2,3-d]pyrimidin-7-one;
4-(butyl-ethyl-amino)-2-methyl-8-(2,6-dimethyl-4-bromo-phenyl)-5,8-dihydro-6H-pyrido[2,3-d]pyrimidin-7-one;
4-(1-ethyl-propoxy)-2-methyl-8-(2,6-dimethyl-4-bromo-phenyl)-5,8-dihydro-6H-pyrido[2,3-d]pyrimidin-7-one;
(butyl-ethyl)-[2-methyl-8-(2,6-dimethyl-4-bromo-phenyl)-5,6,7,8-tetrahydro-pyrido[2,3-d]pyrimidin-4-yl]-amine;
(propyl-ethyl)-[2-methyl-8-(2,6-dimethyl-4-bromo-phenyl)-5,6,7,8-tetrahydro-pyrido[2,3-d]pyrimidin-4-yl]-amine;
(diethyl)-[2-methyl-8-(2,6-dimethyl-4-bromo-phenyl)-5,6,7,8-tetrahydro-pyrido [2,3-d]pyrimidin-4-yl]-amine;
(1-ethyl-propyl)-[2-methyl-8-(2,6-dimethyl-4-bromo-phenyl)-5,6,7,8-tetrahydro-pyrido[2,3-d]pyrimidin-4-yl]-amine;
(1-ethyl-propoxy)-2-methyl-8-(2,6-dimethyl-4-bromo-phenyl)-5,6,7,8-tetrahydro-pyrido[2,3-d]pyrimidine;
4-(butyl-ethyl-amino)-2-methyl-8-(2,4,6-trimethyl-phenyl)-5,8-dihydro-6H-pyrido[2,3-d]pyrimidin-7-one;
4-(1-ethyl-propoxy)-2-methyl-8-(2,4,6-trimethyl-phenyl)-5,8-dihydro-6H-pyrido [2,3-d] pyrimidin-7-one;
(butyl-ethyl)-[2-methyl-8-(2,4,6-trimethyl-phenyl)-5,6,7,8-tetrahydro-pyrido[2,3-d] pyrimidin-4-yl]-amine;
(propyl-ethyl)-[2-methyl-8-(2,4,6-trimethyl-phenyl)-5,6,7,8-tetrahydro-pyrido [2,3-d] pyrimidin-4-yl]-amine;
(diethyl)-[2-methyl-8-(2,4,6-trimethyl-phenyl)-5,6,7,8-tetrahydro-pyrido[2,3-d] pyrimidin-4-yl]-amine;
(1-ethyl-propyl)-[2-methyl-8-(2,4,6-trimethyl-phenyl)-5,6,7,8-tetrahydro-pyrido[2,3-d]pyrimidin-4-yl]-amine;
(1-ethyl-propoxy)-2-methyl-8-(2,4,6-trimethyl-phenyl)-5,6,7,8-tetrahydro-pyrido[2,3-d] pyrimidine;
8-(1-ethyl-propoxy)-6-methyl-4-(2,6-dimethyl-4-bromo-phenyl)-3,4-dihydro-1H-pyrido [2,3-b]pyrazin-2-one;
8-(1-ethyl-propoxy)-6-methyl-4-(2,6-dimethyl-4-bromo-phenyl)-1,2,3,4-tetrahydro-pyrido [2,3-b]pyrazine;
4-(1-ethyl-propoxy)-2-methyl-8-(2,6-dimethyl-4-bromo-phenyl)-quinoline;
5-(1-ethyl-propoxy)-7-methyl-1-(2,6-dimethyl-4-bromo-phenyl)-1,4-dihydro-2H-3-oxa- 1,8-diaza-naphthalene;
5-(1-ethyl-propoxy)-7-methyl-1-(2,6-dimethyl-4-bromo-phenyl)-1,2-dihydro-3-oxa-1, 8-diaza-naphthalen-4-one;
8-(1-ethyl-propoxy)-1,6-dimethyl-4-(2,6-dimethyl-4-bromo-phenyl)-1,2,3,4-tetrahydro- pyrido[2,3-b]pyrazine;
(1-ethyl-propyl)-[2-methyl-8-(2,6-dimethyl-4-bromo-phenyl)-quinolin-4-yl]-amine;
4-(butyl-ethyl-amino)-2,6-dimethyl-8-(2,6-dimethyl-4-chloro-phenyl)-5,8-dihydro-6H-pyrido[2,3-d]pyrimidin-7-one;
8-(1-ethyl-propoxy)-6-methyl-4-(2,6-dimethyl-4-chloro-phenyl)-3,4-dihydro-1H-pyrido[2,3-b]pyrazin-2-one;
8-(1-ethyl-propoxy)-6-methyl-4-(2,6-dimethyl-4-chloro-phenyl)-1,2,3,4-tetrahydro-pyrido[2,3-b]pyrazine;
4-(1-ethyl-propoxy)-2-methyl-8-(2,6-dimethyl-4-chloro-phenyl)-quinoline;
5-(1-ethyl-propoxy)-7-methyl-1-(2,6-dimethyl-4-chloro-phenyl)-1,4-dihydro-2H-3-oxa- 1,8-diaza-naphthalene;
5-(1-ethyl-propoxy)-7-methyl-1-(2,6-dimethyl-4-chloro-phenyl)-1,2-dihydro-3-oxa-1,8- diaza-naphthalen-4-one;
8-(1-ethyl-propoxy)-1,6-dimethyl-4-(2,6-dimethyl-4-chloro-phenyl)-1,2,3,4-tetrahydro- pyrido[2,3-b]pyrazine;
(1-ethyl-propyl)-[2-methyl-8-(2,6-dimethyl-4-chloro-phenyl)-quinolin-4-yl]-amine;
8-(1-hydroxymethyl-propoxy)-6-methyl-4-(2,4,6-trimethyl-phenyl)-3,4-dihydro-1H-pyrido[2,3-b]pyrazin-2-one;
8-(1-hydroxymethyl-propylamino)-6-methyl-4-(2,4,6-trimethyl-phenyl)-3,4-dihydro-1H-pyrido[2,3-b]pyrazin-2-one ;
8-(1-ethyl-propylamino)-6-methyl-4-(2,4,6-trimethyl-phenyl)-3,4-dihydro-1H-pyrido [2,3-b]pyrazin-2-one;
8-diethylamino-6-methyl-4-(2,4,6-trimethyl-phenyl)-3,4-dihydro-1H-pyrido[2,3-b] pyrazin-2-one;
8-(ethyl-propyl-amino)-6-methyl-4-(2,4,6-trimethyl-phenyl)-3,4-dihydro-1H-pyrido[2,3-b]pyrazin-2-one ;
8-(butyl-ethyl-amino)-6-methyl-4-(2,4,6-trimethyl-phenyl)-3,4-dihydro-1H-pyrido [2,3-b]pyrazin-2-one;
8-(1-hydroxymethyl-propoxy)-6-methyl-4-(2,4,6-trimethyl-phenyl)-1,2,3,4-tetrahydro-pyrido[2,3-b]pyrazine;
8-(1-hydroxymethyl-propylamino)-6-methyl-4-(2,4,6-trimethyl-phenyl)-1,2,3,4-tetrahydro-pyrido[2,3-b]pyrazine;
8-(1-ethyl-propylamino)-6-methyl-4-(2,4,6-trimethyl-phenyl)-1,2,3,4-tetrahydro-pyrido[2,3-b]pyrazine;
8-diethylamino-6-methyl-4-(2,4,6-trimethyl-phenyl)-1,2,3,4-tetrahydro-pyrido [2,3-b]pyrazine;
8-(ethyl-propyl-amino)-6-methyl-4-(2,4,6-trimethyl-phenyl)-1,2,3,4-tetrahydro-pyrido[2,3-b]pyrazine;
8-(butyl-ethyl-amino)-6-methyl-4-(2,4,6-trimethyl-phenyl)-1,2,3,4-tetrahydro-pyrido[2,3-b]pyrazine;
4-(1-hydroxymethyl-propoxy)-2-methyl-8-(2,4,6-trimethyl-phenyl)-quinoline;
4-(1-hydroxymethyl-propylamino)-2-methyl-8-(2,4,6-trimethyl-phenyl)-quinoline;
4-(1-ethyl-propylamino)-2-methyl-8-(2,4,6-trimethyl-phenyl)- quinoline;
4-diethylamino-2-methyl-8-(2,4,6-trimethyl-phenyl)- quinoline;
4-(ethyl-propyl-amino)-2-methyl-8-(2,4,6-trimethyl-phenyl)- quinoline;
4-(butyl-ethyl-amino)-2-methyl-8-(2,4,6-trimethyl-phenyl)- quinoline;
5-(1-hydroxymethyl-propoxy)-7-methyl-1-(2,4,6-trimethyl-phenyl)-1,4-dihydro-2H-3-oxa-1,8-diaza-naphthalene;
5-(1-hydroxymethyl-propylamino)-7-methyl-1-(2,4,6-trimethyl-phenyl)-1,4-dihydro-2H-3-oxa-1,8-diaza-naphthalene;
5-(1-ethyl-propylamino)-7-methyl-1-(2,4,6-trimethyl-phenyl)-1,4-dihydro-2H-3-oxa-1,8-diaza-naphthalene;
5-diethylamino-5-methyl-1-(2,4,6-trimethyl-phenyl)-1,4-dihydro-2 H-3-oxa-1,8-diaza-naphthalene;
5-(ethyl-propyl-amino)-7-methyl-1-(2,4,6-trimethyl-phenyl)-1,4-dihydro-2H-3-oxa-1,8-diaza-naphthalene; and
8-(butyl-ethyl-amino)-6-methyl-4-(2,4,6-trimethyl-phenyl)-1,4-dihydro-2H-3-oxa-1,8-diaza-naphthalene.

The invention also relates to a pharmaceutical composition for the treatment, prevention or inhibition of (a) a disorder the treatment of which can be effected or facilitated by antagonizing CRF, including but not limited to disorders induced or facilitated by CRF, or (b) a disorder selected from inflammatory disorders such as rheumatoid arthritis and osteoarthritis, pain, asthma, psoriasis and allergies; generalized anxiety disorder; panic; phobias; obsessive-compulsive disorder; post-traumatic stress disorder; sleep disorders induced by stress; pain perception such as fibromyalgia; mood disorders such as depression, including major depression, single episode depression, recurrent depression, child abuse induced depression, mood disorders associated with premenstrual syndrome, and postpartum depression; dysthemia; bipolar disorders; cyclothymia; chronic fatigue syndrome; stress-induced headache; cancer; irritable bowel syndrome, Crohn's disease; spastic colon; post operative ileus; ulcer; diarrhea; stress-induced fever; human immunodeficiency virus (HIV) infections; neurodegenerative diseases such as Alzheimer's disease, Parkinson's disease and Huntington's disease; gastrointestinal diseases; eating disorders such as anorexia and bulimia nervosa; hemorrhagic stress; chemical dependencies and addictions (e.g., dependencies on alcohol, nicotine, cocaine, heroin, benzodiazepines, or other drugs); drug and alcohol withdrawal symptoms; stress-induced psychotic episodes; euthyroid sick syndrome; syndrome of inappropriate antidiarrhetic hormone (ADH); obesity; infertility; head traumas; spinal cord trauma; ischemic neuronal damage (e.g., cerebral ischemia such as cerebral hippocampal ischemia); excitotoxic neuronal damage; epilepsy; stroke; immune dysfunctions including stress induced immune dysfunctions (e.g., porcine stress syndrome, bovine shipping fever, equine paroxysmal fibrillation, and dysfunctions induced by confinement in chickens, sheering stress in sheep or human-animal interaction related stress in dogs); muscular spasms; urinary incontinence; senile dementia of the Alzheimer's type; multiinfarct dementia; amyotrophic lateral sclerosis; hypertension; tachycardia; congestive heart failure; osteoporosis; premature birth; and hypoglycemia in a mammal, including a human, comprising an amount of a compound of the formula I, or a pharmaceutically acceptable salt thereof, that is effective in the treatment of such disorder, and a pharmaceutically acceptable carrier.

The invention also relates to a method for the treatment, prevention or inhibition of (a) a disorder the treatment of which can be effected or facilitated by antagonizing CRF, including but not limited to disorders induced or facilitator by CRF, or (b) a disorder selected from inflammatory disorders such as rheumatoid arthritis and osteoarthritis, pain, asthma, psoriasis and allergies; generalized anxiety disorder; panic; phobias; obsessive-compulsive disorder; post-traumatic stress disorder; sleep disorders induced by stress; pain perception such as fibromyalgia; mood disorders such as depression, including major depression, single episode depression, recurrent depression, child abuse induced depression, mood disorders associated with premenstrual syndrome, and postpartum depression; dysthemia; bipolar disorders; cyclothymia; chronic fatigue syndrome; stress-induced headache; cancer; irritable bowel syndrome; Crohn's disease; spastic colon; post operative ileus; ulcer; diarrhea; stress-induced fever; human immunodeficiency virus (HIV) infections; neurodegenerative diseases such as Alzheimer's disease, Parkinson's disease and Huntington's disease; gastrointestinal diseases; eating disorders such as anorexia and bulimia nervosa; hemorrhagic stress; stress-induced psychotic episodes; euthyroid sick syndrome; syndrome of inappropriate antidiarrhetic hormone (ADH); obesity; infertility; head traumas; spinal cord trauma; ischemic neuronal damage (e.g., cerebral ischemia such as cerebral hippocampal ischemia); excitotoxic neuronal damage; epilepsy; stroke; immune dysfunctions including stress induced immune dysfunctions (e.g., porcine stress syndrome, bovine shipping fever, equine paroxysmal fibrillation, and dysfunctions induced by confinement in chickens, sheering stress in sheep or human-animal interaction related stress in dogs); muscular spasms; urinary incontinence; senile dementia of the Alzheimer's type; multiinfarct dementia; amyotrophic lateral sclerosis; chemical dependencies and addictions (e.g., dependencies on alcohol, nicotine, cocaine, heroin, benzodiazepines, or other drugs); drug and alcohol withdrawal symptoms; hypertension; tachycardia; congestive heart failure; osteoporosis; premature birth; and hypoglycemia in a mammal, including a human, comprising administering to a subject in need of said treatment an amount of a compound of the formula I, or a pharmaceutically acceptable salt thereof, that is effective in treating such disorder.

This invention also relates to a method of treating or preventing a disorder or condition, the treatment or prevention of which can be effected or facilitated by inhibiting CRH binding protein, in a mammal, including a human, comprising administering to said mammal a CRH binding protein inhibiting amount of a compound of the formula I, or a pharmaceutically acceptable salt thereof.

This invention also relates to a pharmaceutical composition for treating or preventing a disorder or conditon, the treatment or prevention of which can be effected or facilitated by inhibiting CRH binding protein in a mammal, including a human, comprising a CRH binding protein inhibiting amount of a compound of the formula I, or a pharmaceutically acceptable salt thereof, and a pharmaceutically acceptable carrier.

This invention includes all optical isomers and other stereoisomers of compounds of the formula I. When such compounds contain one or more chiral centers, it is understood that the invention includes the racemic mixtures as well as all individual enantiomers and diastereomers of such compounds, and mixtures thereof.

The compounds of this invention include compounds identical to those described above but for the fact that one or more hydrogen, nitrogen or carbon atoms are replaced by isotopes thereof (e.g., tritium or carbon-14 isotopes). Such compounds are useful as research and diagnostic tools in metabolism pharmokinetic studies and in binding assays.

### Detailed Description of the Invention

The following compounds having the formulas II through V are useful as intermediates in the synthesis of compounds of the formula I. 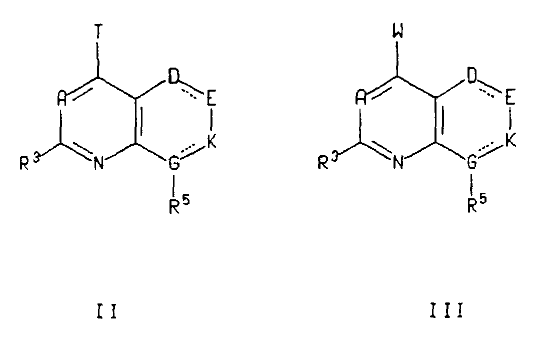 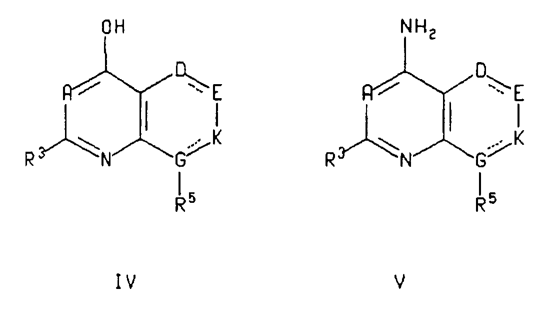 In the above compounds of formulas II-V, T is chloro, bromo, iodo or -OSO₂CF₃; W is cyano, -CHO, or -COO(C₀-C₄ alkyl), and A, D, E, K, G, R³, and R⁵ are as defined above with reference to formula I.

Methods of preparing the compounds and compositions of this invention are described below. In the discussion and reaction schemes that follow, R¹ through R¹³, A, B, D, E, K, G, Z, Z², T and W, the dashed lines and structural formulas I, II, III, IV and V, unless otherwise indicated, are defined as above.

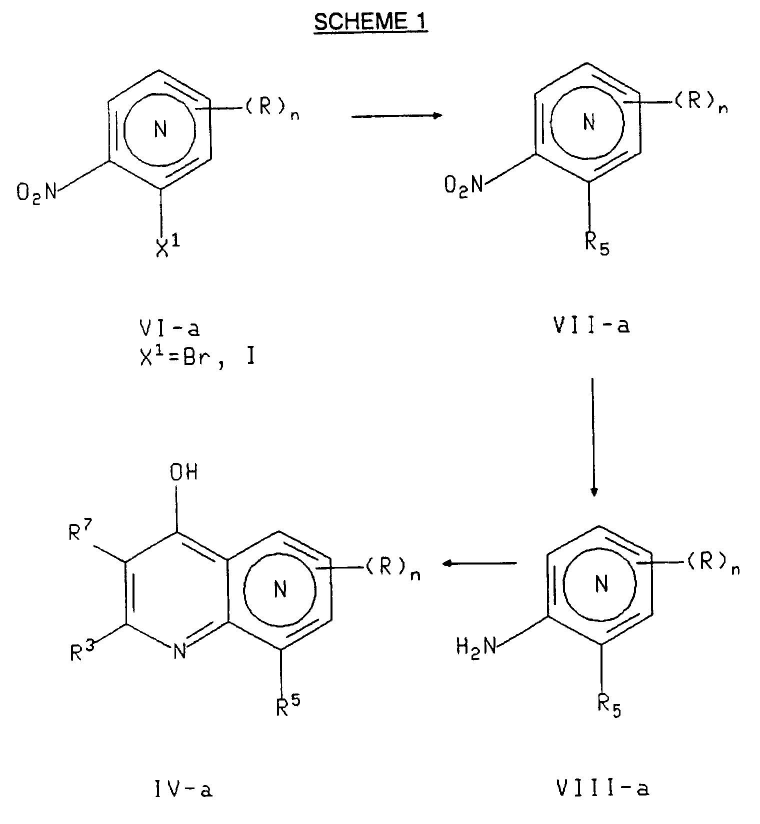

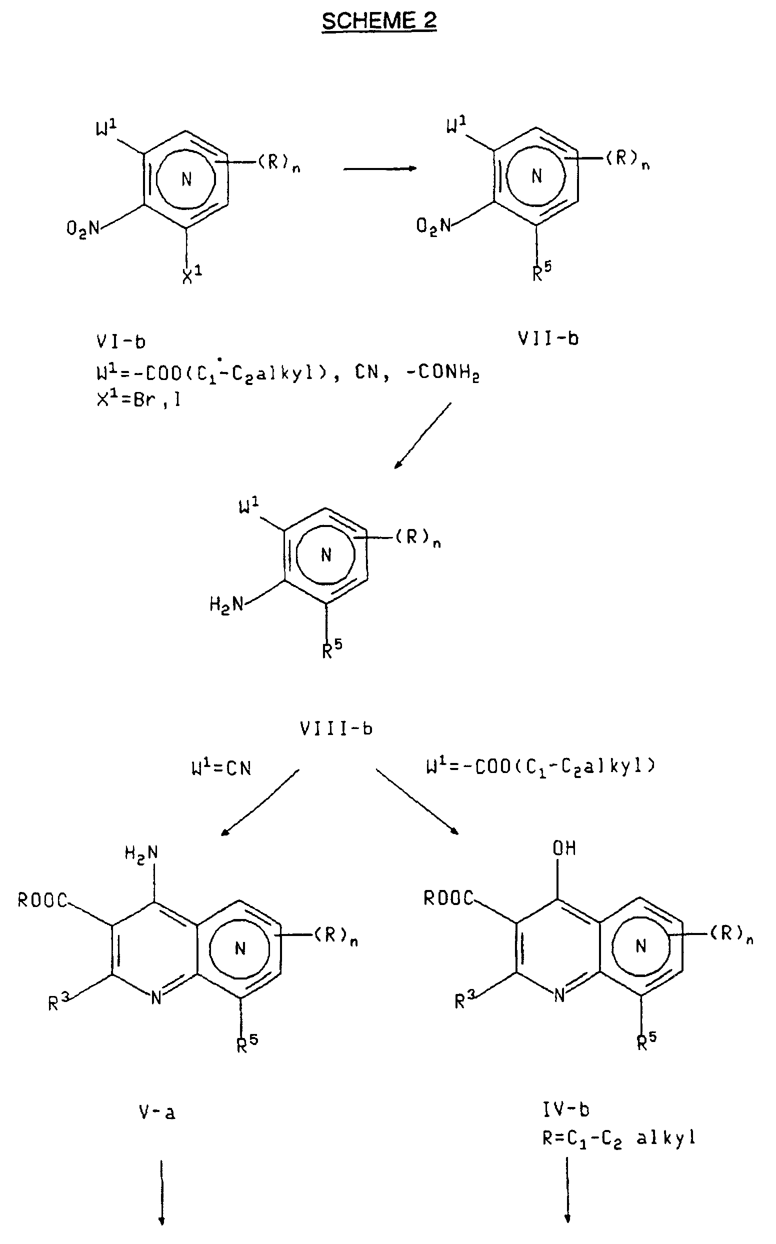

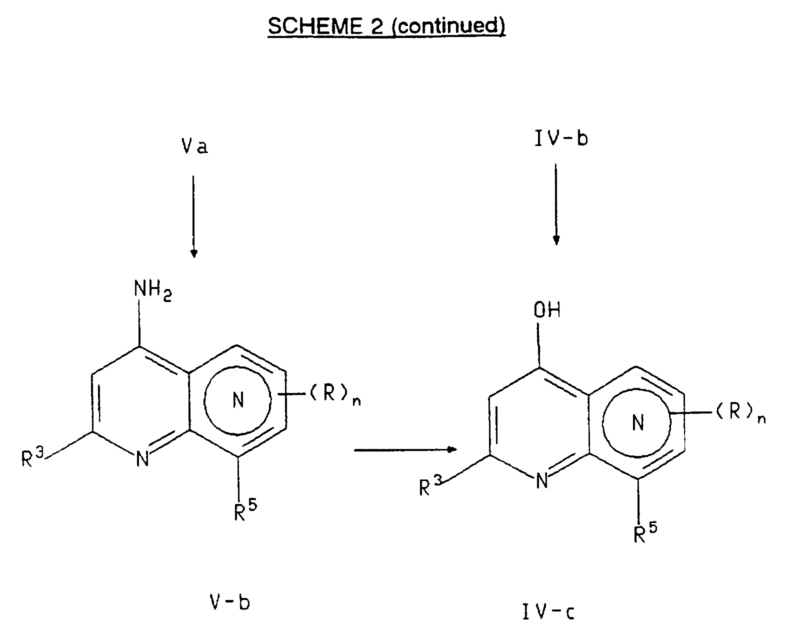

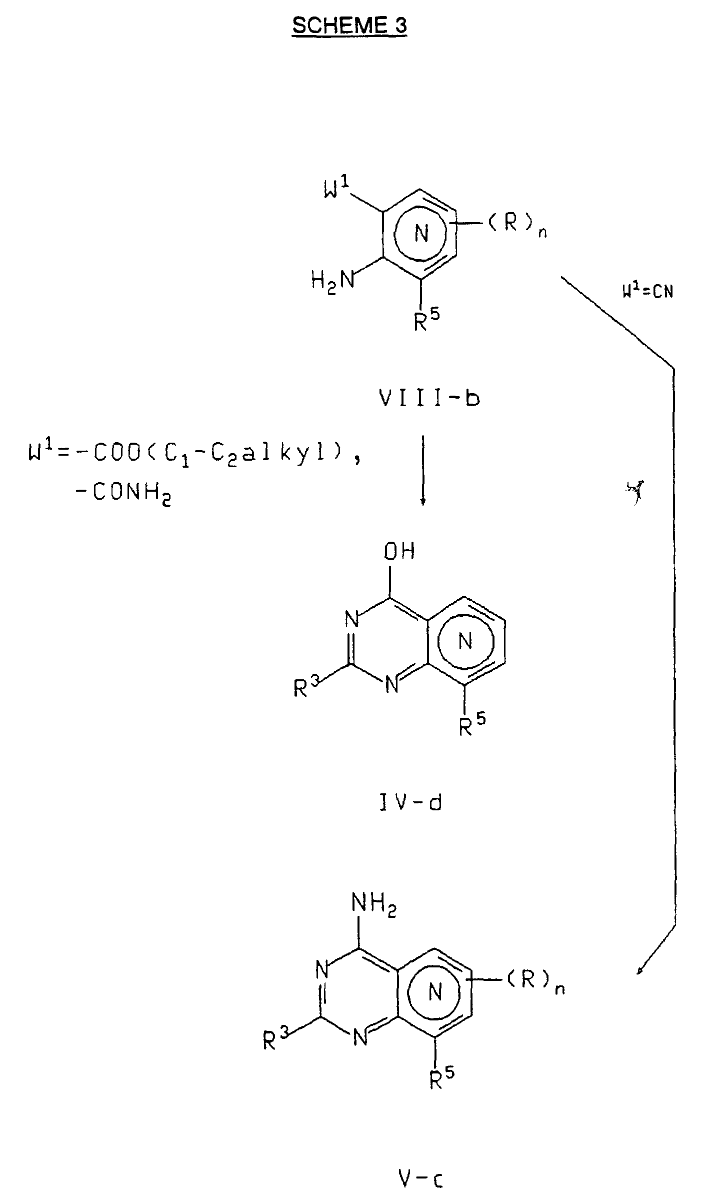

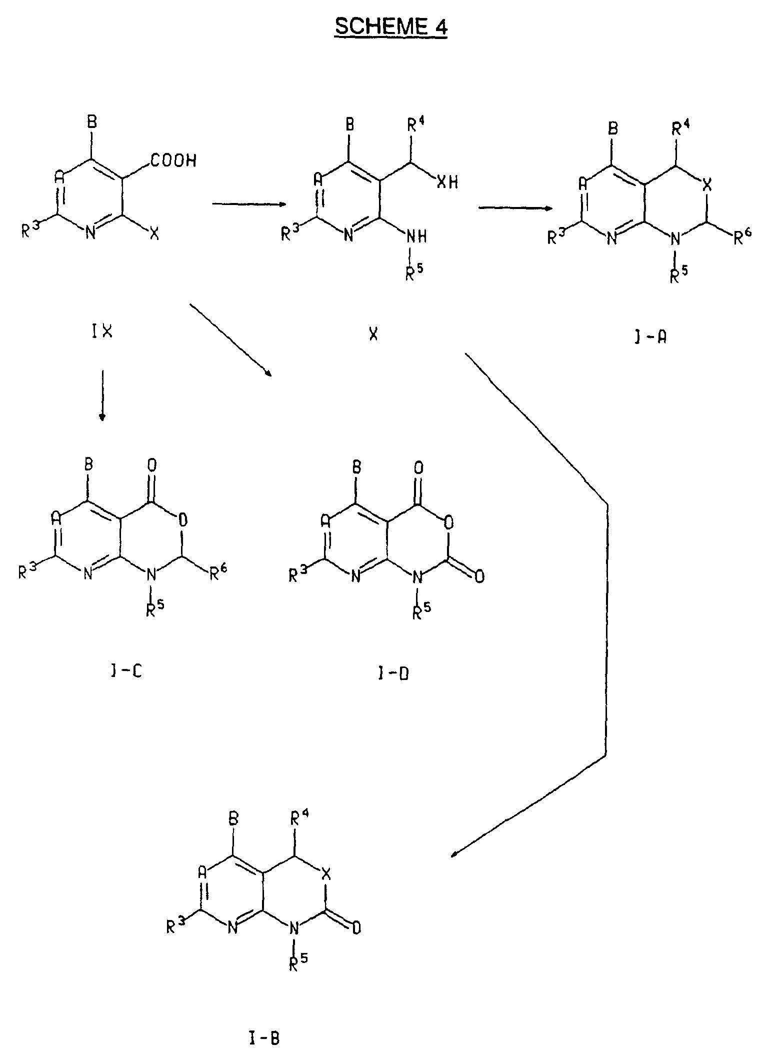

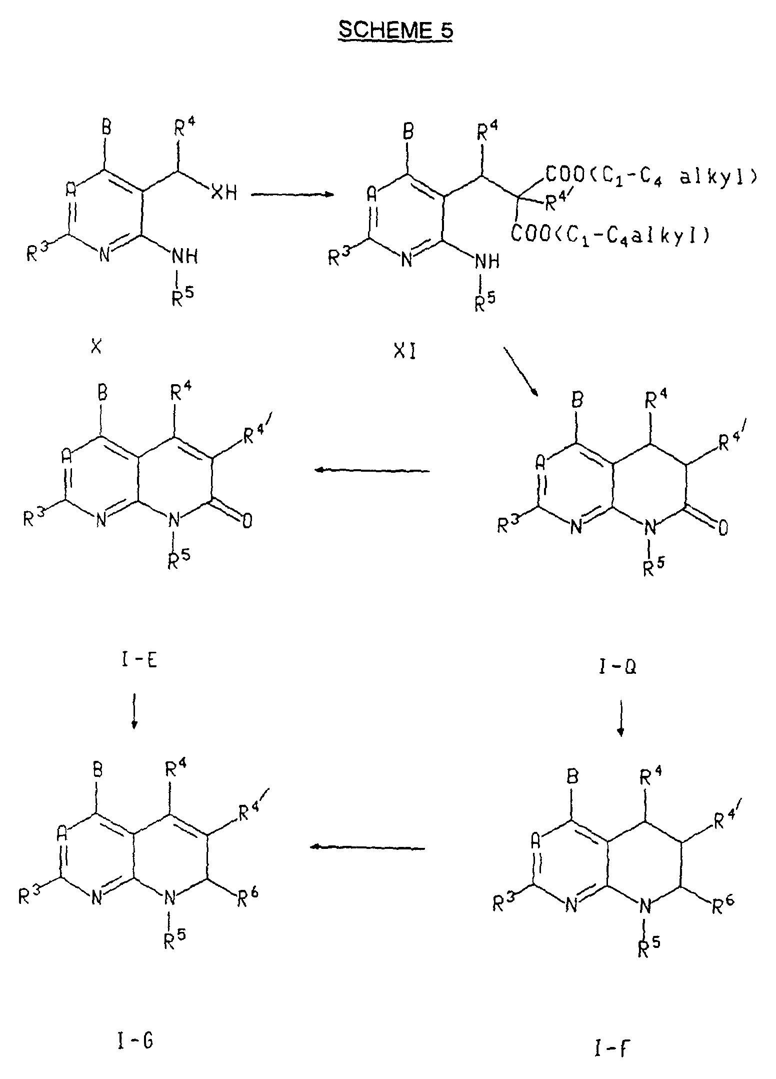

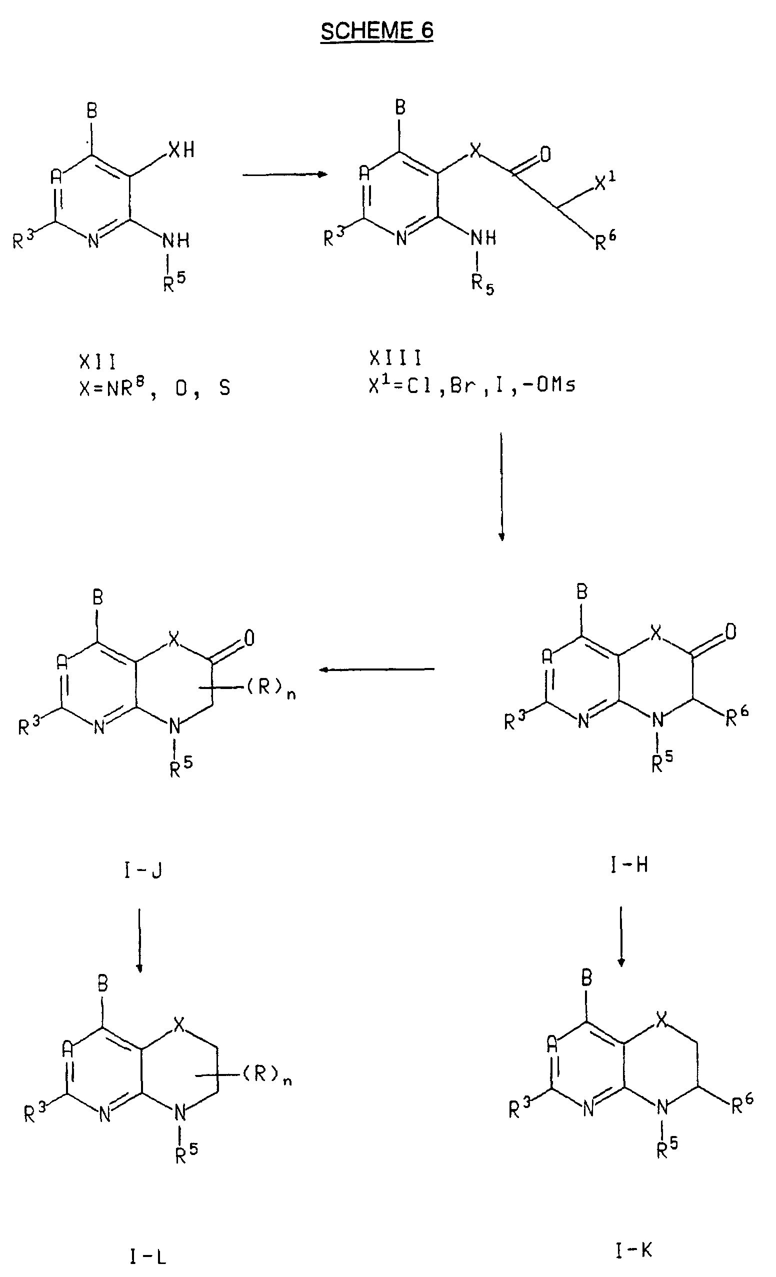

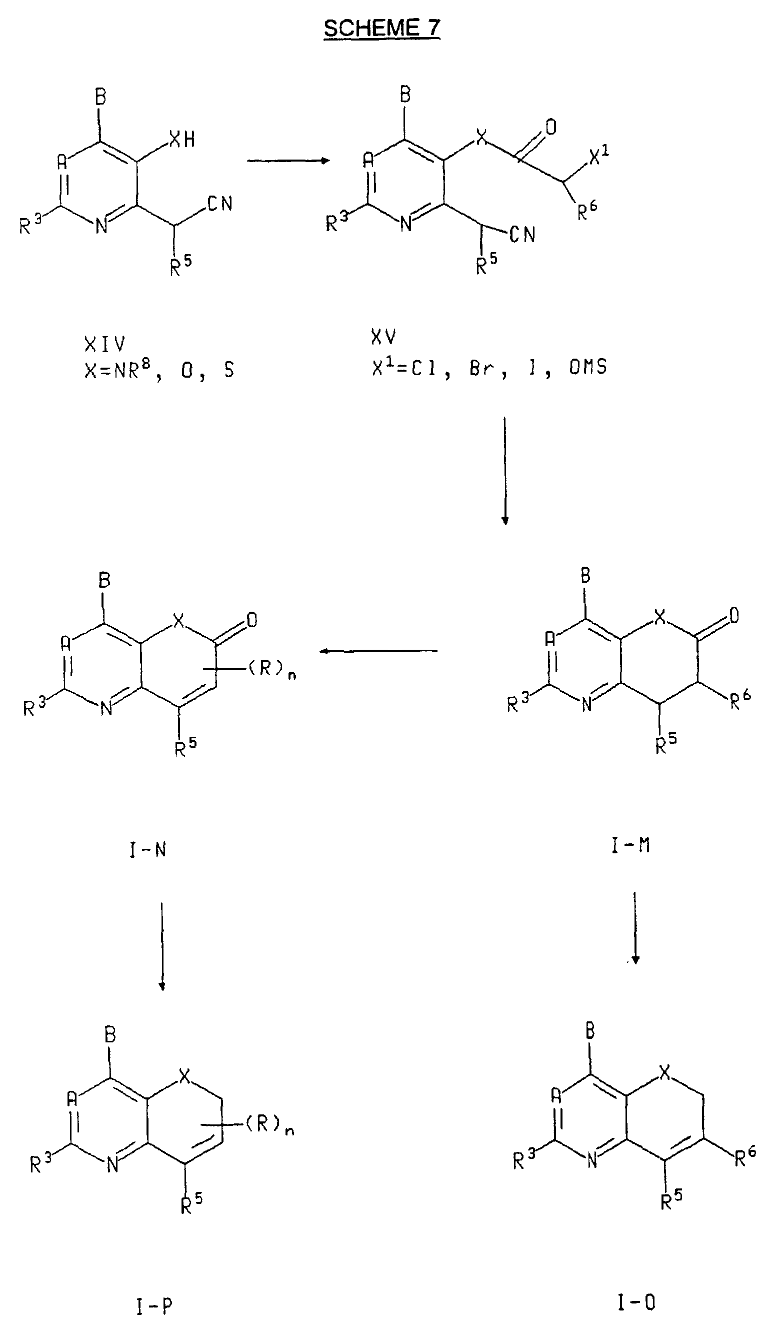

Compounds of the formula I wherein B is -NR¹R² or -NHCR¹R²R¹¹ may be prepared by reacting a compound of the formula II wherein T is chloro, bromo, or iodo with a compound of the formula BH, in the presence of a base, with or without an organometallic compound such as Cu(I)X, wherein X is chloro, bromo or iodo, or an acid (such as p-TsOH (Ts=Tosyl) or another sterically hindered phenol) or an equivalent agent known to those of skill in the art. Suitable solvents for this reaction include DMSO, NMP and THF. An excess of BH may be used as both the reagent and the base. Other bases such as potassium or sodium carbonate, a trialkylamine, a potassium or sodium (C₁-C₄ alkoxide) or sodium hydride may also be used. When R⁷ is an electron withdrawing group such as -COO(C₁-C₄alkyl) or CN, the reaction generally is carried out at a temperature between about room temperature and about 130°C. When R⁷ is a non-electron withdrawing group, the reaction temperature can generally range from about 50°C to about 270°C and the pressure can generally range from about 27579 Pa (4 psi) to about 2068428 Pa (300 psi). A pressure reactor may be used.

Alternatively, the compounds of formula I may be prepared by reacting a compound of the formula II wherein T is bromo or iodo with 1 equivalent or an excess of BH and a base such as sodium or potassium carbonate or a sodium or potassium (C₁-C₄ alkoxide), in the presence of a palladium (II) or a palladium (0) catalyst such as Pd(OAc)₂ or Pd(PPh₃)₄, together with a racemic or chiral phosphino agent such as 2,2-bis(diphenylphosphino)-1,1-binaphthyl (BINAP). Alternatively, premade Pd(II)(BINAP) may be used directly in an appropriate inert (i.e., inert with respect to the reaction at hand) solvent such as toluene, xylene, or dioxane or sulfolane, at a temperature from about room temperature to about 180°C, preferably at about reflux temperature.

Compounds of the formula I wherein B is -OCR¹R²R¹¹, -SCR¹R²R¹¹, or
-NHCR¹R²R¹¹ may be prepared by reacting compounds in the formula II wherein T is chloro, bromo or iodo with a compound of the formula BH in the presence of a base which is capable of deprotonation of BH (e.g., sodium or potassium hydride, or an organometallic base such as sodium diisopropylamide, sodium bis(trimethylsilyl)amide, lithium diisopropylamide, lithium bis(trimethylsilyl)amide, a sodium C₁-C₄ alkoxide or n-butyllithium), in an appropriate inert solvent such as tetrahydrofuran, acetonitrile, dimethylsulfoxide, acetone, a C₂-C₅ alcohol, chloroform, benzene, xylene, toluene, N,N-dimethylformamide (DMF), methylene chloride, 1-methyl-2-pyrrolidinone (NMP) or a mixture of two or more of the above solvents (e.g., DMSO and THF), at a temperature from about 0°C to about 180°C, preferably from about 50°C to about 180°C.

Compounds of the formula I wherein B is -CR¹R²R¹¹, -C(C=CR²R¹²)R¹, -CR²R¹¹NHR¹, -CR²R¹¹OR¹, -CR²R¹¹SR¹ or -C(O)R² may be prepared from compounds of the formula III wherein W is cyano, formyl or carboxy, as described below.

Reacting compounds of formula III wherein W is cyano with a Grignard reagent containing the group R² will yield the corresponding compounds of formula I wherein B is -COR². Further reaction of the compounds of formula I wherein B is COR² with a Grignard reagent containing R¹ will yield the corresponding the compounds of formula I wherein B is -CR¹R²OH. Reacting compounds of formula III wherein W is formyl with a Grignard reagent containing the group R² will yield the corresponding compounds of the formula I wherein B is -CHR²OH. Suitable solvents for the above Grignard reactions include ethereal solvents such as THF, ether, dioxane and glyme.

Compounds of formula I wherein B is -CR¹R²R¹¹ or -C(C=CR²R¹¹)R¹ may be prepared by conventional methods. Thus, reaction of a compound of the formula I wherein B is -CR¹'R²'OH, (wherein R¹' and R²' are defined as R¹ and R², respectively, except that R¹' may not be R¹ and R²' may not be R²), with an acid such as concentrated sulfuric acid in acetic acid, or a Burgess inner salt such as (carboxysulfamoyl)triethylammonium hydroxide methyl ester, will yield a compound of the formula I wherein B is -C(=CR²R¹¹)R¹. Hydrogenation of a compound of formula I wherein B is -C(=CR²R¹¹)R¹ using palladium on carbon (Pd/C) or a platinum oxide catalyst in a C₁-C₄ alkanol solvent, ethyl acetate, benzene or THF will yield a compound of the formula I wherein B is -CHR¹R². Reaction of a compound of the formula I wherein B is -CR¹R²OH with diethylaminosulfur trifluoride or triphenylphosphine/carbon tetrachloride in an inert organic solvent such as carbon tetrachloride will afford a compound of the formula I wherein B is -CR¹R²F or -CR¹R²Cl, respectively.

Reduction of a compound of formula I wherein B is -COR² with sodium borohydride in an appropriate inert solvent such as a C₁-C₄ alkanol will yield a compound of the formula I wherein B is -CHR²OH. Alkylation of a compound of the formula I wherein B is -CHR²OH with an alkyl halide (such as alkyl iodide) in the presence of a base such as sodium hydride (NaH) at about room temperature, in an inert organic solvent such as DMF, ether, DMSO, dioxane, or THF, will yield the corresponding compound of the formula I wherein B is -CHR²OR¹.

Compounds of the formula I wherein B is -CR²R¹⁰NHR¹ may be prepared by conventional methods such as reductive amination of the corresponding compounds of the formula I wherein B is -C(O)R² with an appropriate amine and reducing agent (such as sodium cyanoborohydride, sodium triacetoxyborohydride or lithium aluminum tetrahydride) in an appropriate inert solvent such as a C₁-C₄ alkanol or acetic acid.

Conversion of compounds in formula I wherein B is -C(O)R² into compounds in formula I wherein B is -C(S)R² can be accomplished using standard methods well known in the art (e.g., using Lawesson's Reagent or diphosphorus pentasulfide (P₂S₅)). Reduction of compounds of the formula I wherein B is -C(S)R² with a reducing agent such as sodium borohydride in a (C₁-C₄) alkanol or lithium aluminum tetrahydride in THF or ether, at a temperature from about room temperature to about the reflux temperature, gives the corresponding compounds of the formula I wherein B is - CHR²SH. Alkylation of compounds of the formula I wherein B is -CHR²SH with an alkyl halide (such as alkyl iodide) in the presence of a base such as sodium hydride in such an inert solvent such as DMF, at a temperature from about room temperature to about the reflux temperature will afford the corresponding compounds of the formula I wherein B is -CHR²SR¹.

Compounds in formula II may be prepared from compounds of the formula IV or V as described below.

Compounds of formula II wherein T is chloro, bromo or iodo can be prepared by reacting compounds of the formula IV with from one equivalent to an excess of POT₃ wherein T is chloro, bromo or iodo, in the presence or absence of a di(C₁-C₄ alkyl)aniline, preferably diethylaniline, with or without a solvent (such as dichloroethane, DMF, dimethylsulfoxide (DMSO) or acetamide), at a temperature from about room temperature to about 180°C, preferably from about 100°C to about 150°C. Alternatively, compounds of formula II wherein T is chloro, bromo or iodo can be prepared by reacting the corresponding compounds of formula II wherein T is - OSO₂CF₃ with a sodium or potassium halide in an appropriate inert solvent such as sulfolane, DMSO, DMF, or acetonitrile, at a temperature from about 60°C to about 180°C. Compounds of formula II wherein T is -OSO₂CF₃ can be prepared by reacting compounds of formula IV with Tf₂O in the presence of a base such as triethylamine or pyridine, in an appropriate inert solvent such as THF, methylene chloride, dioxane, ether or toluene, at a temperature from about 0°C to about 50°C, preferably from about 0°C to about room temperature.

Alternatively, compounds of formula II wherein T is chloro, bromo or iodo may be prepared by reacting compounds of formula V with a (C₁-C₇ alkyl)-nitrite and Cu(I)T₂ (wherein T is chloro, bromo or iodo) in an appropriate inert solvent such as acetonitrile, acetone, methylene chloride, THF, dioxane, benzene, toluene, dichloroethane, DMF, DMSO or N-methylpyrrolidinone (NMP) at a temperature from about room temperature to about 150°C, preferably from about 40°C to about 100°C.

Compounds of formula III wherein W is cyano can be prepared by reacting the corresponding compounds of formula II wherein T is chloro, bromo or iodo with potassium cyanide, copper cyanide, sodium cyanide or a di(C₁-C₄alkyl)aluminum cyanide in an appropriate inert solvent such as dimethylsulfoxide, DMF, toluene or xylene, at temperature from about room temperature to about 180°C, preferably from about 60°C to about 150°C, with or without Pd(II)OAc or Pd(O)(PPh₃)₄.

Compounds of formula III wherein W is -CHO or -COOH may be prepared by reacting compounds in formula II wherein T is bromo or iodo with an organolithium reagent such as t-BuLi, s-BuLi, or n-BuLi in an appropriate inert solvent such as THF, dioxane, ether, benzene or methylene chloride, at temperature from about -120°C to about room temperature, preferably from about -110°C to about -60°C, followed by quenching with an appropriate electrophile such as DMF or CO₂ (gas or dry ice), to give compounds of formula III wherein W is -CHO and -COOH, respectively.

It is understood that the general organic chemistry knowledge can be applied to all the cases in which one of the reaction sequences referred to herein can be changed. Changing the reaction sequence is based on the feasibility of a particular reaction at a particular step in a sequence, such as using a protecting group at any stage of a synthesis that is workable, or reducing an ester group to the corresponding C₁-C₄ alkyl group at any convenient stage of a synthesis. Compounds of formula I wherein R³ is bromo, chloro, -COO(C₁-C₄ alkyl) or -COOH may be converted to the corresponding compounds wherein R³ is (C₁-C₄ alkyl), -O(C₁-C₄ alkyl), F or -S(C₁-C₄ alkyl) by methods described in the literature. This conversion may not need to be done at the last stage of a particular synthesis, but rather may be more conveniently performed at an earlier stage.

Compounds of formula I or other formulas described herein wherein R³ is -O-(C₁-C₄ alkyl) or -S(C₁-C₄ alkyl) may be prepared by reacting the corresponding compounds wherein R³ is chloro, bromo or iodo with a nucleophile such as a C₁-C₄ alkanol or a C₁-C₄ alkanethiol with an organic or inorganic base. Suitable bases for this reaction include sodium and sodium hydride. Compounds of formula I or any of the other formulas described herein wherein R³ is fluoro may be prepared by reacting the corresponding compounds wherein R³ is chloro with tetrabutylammonium fluoride in a suitable inert solvent such as DMSO, methylene chloride or tetrahydrofuran. Tetrahydrofuran is preferred. The reaction temperature can range from about room temperature to about 180°C. Reduction of compound wherein R³ is an ester using LiAlH₄/AlCl₃ in an appropriate inert solvent such as THF, ether, or dioxane, at temperature from about room temperature to about 100°C, affords the corresponding compound wherein R³ is methyl. Conversion of compounds wherein B is -COOH to the corresponding compounds wherein B is -CO(C₁-C₃ alkyl) may be performed using methods well known in art. Reduction of compounds wherein B is -CO(C₁-C₃ alkyl) using standard literature methods will afford compounds wherein R³ is one of a variety of (C₁-C₄ alkyl) derivatives.

Compounds of formula IV-a, wherein the right hand side of the six membered ring represents a benzo, pyrido, pyrimido, or pyridazino ring, (R)ₙ represents from zero to three substituents as defined in formula IV, and R³, R⁵ and R⁷ are as defined above with reference to formula IV, may be prepared, as shown in Scheme 1, starting from compounds of formula VI-a, wherein the 6-membered ring represents a benzo, pyrido, pyrimido, or pyridazino ring, (R)ₙ represents from zero to three substituents that are the substituents previously defined for the compounds of the formula IV, and X¹ is Br or I. Compounds of formula VII-a may be prepared using Suzuki coupling, Stille coupling or Ullman biaryl synthesis, as described in the literature (See Tetrahedron Lett., 37, 1043-1044, 1996; Tetrahedron, 36, 3111-4, 1995; J. Chem.Soc. Chem. Commun., 2551-2553, 1995; J. Org. Chem., 49, 5237-5243, 1984; Synlett, 765-766, 1995; Synlett, 207, 1992; ). Examples of suitable reaction conditions are: (a) reacting a compounds of the formula VI-a wherein X¹ is Br or I with R⁵-B(OH)₂ and a base such as aqueous sodium carbonate, aqueous sodium hydroxide, Ba(OH)₂, Cs₂CO₃, K₃PO₄, 10% TIOH, sodium or potassium (C₁-C₄ alkoxide), in the presence of catalytic amount (0.5 mol% to 50% mol%) of a Pd(0) or Pd(II) compound, together with racemic or a chiral phosphino ligand, preferably Pd(PPh₃)₄, in an appropriate inert solvent such as dimethoxyethane (DME), N,N-dimethylformamide (DMF), benzene, dimethylacetamide (DMA), a C₁-C₆ alkanol such as ethanol, dioxane, N-methylpyrrolidinone (NMP) or dioxane, at temperature from about 25°C to about 150°C, preferably from about room temperature to about 120°C.

Alternatively, compounds of formula VII-a may be prepared using methods described in the literature (See Tetrahedron, 49, 49-64, 1993; Chem. Ber. 93, 2479-2484,1960; Can. J. Chem., 38, 1445, 1960; Can. J. Chem., 38, 2152-2158, 1960; Pol. J. Chem., 66, 801-805, 1992; Chem. Pharm. Bull., 31, 3460-3464, 1983).

Compounds of formula VIII-a may be prepared using known methods for reducing a nitro group to an amino group. The preferred method is hydrogenation using 5-10% palladium on carbon (Pd/C), at a pressure from about 14 psi to about 55 psi, at about room temperature, in an inert solvent such as ethyl acetate, benzene, THF, or a C₁-C₄ alkanol.

Compounds of formula IV-a may be prepared by heating compounds of formula VIII-a of compound of the formula R³-C(O)-CH(R⁷)-COO(C₁-C₂ alkyl), in the presence of an acid or Lewis acid, with or without a solvent. Examples of such reaction conditions are: a) heating in polyphosphoric acid; b) heating in toluene, benzene or xylene in the presence of acid catalyst (such as p-TsOH, sulfuric acid, HCl(g)) using Dean-Stark trap apparatus; and c) heating in an appropriate solvent such as dichloroethane, Ph₂O or Dowtherm A in the presence of a Lewis acid such as SnCl₄, ZnCl₂/HCl or AlCl₃.

Compounds of formula IV-b and V-a, wherein the right hand side of the six membered ring represents a benzo, pyrido, pyrimido, or pyridazino ring, (R)ₙ is from zero to three substituents as defined in formula IV, and R³, R⁵ and R⁷ are as defined above with reference to formula IV, may be prepared, as shown in Scheme 2, starting from compounds of formula VI-b, wherein the 6-membered ring represents a benzo, pyrido, pyrimido, or pyridazino ring, (R)ₙ is from zero to three substituents which are the substituents previously defined for the compounds in formula IV, X¹ is Br or I and W¹ is CN, -CONH₂ or -COO(C₁-C₂ alkyl). Conversion of compounds of formula VI-b to VIII-b may be performed by the methods analogous to those described above for the conversion of compounds of the formula VI-a into those of the formula VIII-a. Compounds of the formulas IV-b and V-a may be prepared by heating compounds of formula VIII-b wherein W¹ is -COO(C₁-C₂ alkyl) and CN, respectively, with an appropriate R³C(O)CH₂COO(C₁-C₄ alkyl) in the presence of a Lewis acid such as SnCl₄, AlCl₃, TiCl₃ or ZnCl₂, in dichloroethane, at reflux, as illustrated in Scheme 2. Base hydrolysis of IV-b and V-a with sodium hydroxide in H₂O/(C₁-C₄ alcohol) at reflux or with lithium hydroxide in water/THF or water/dioxane at temperature between room temperature to reflux, followed by decarboxylation by heating in an oil bath at a temperature from about 140°C to about 180°C, to give compounds of formula IV-c and V-b, respectively,

Compounds of formula IV-d may be prepared, as shown in Scheme 3, by reacting compounds of formula VIII-b, wherein W¹ is -COO(C₁-C₂ alkyl) or -CONH₂, with (R³CO)₂O or R³COOH or R³C(OC₁-C₂ alkyl)₃ in acetic acid or in an appropriate inert organic solvent such as toluene, dioxane, acetonitrile, methylene chloride or chloroform, at a temperature from between 25°C to about 150°C, preferably at reflux, followed by heating in 85% phosphoric acid or an aqueous acid about such as acetic acid, hydrochloric acid or sulfuric acid, preferably 50-85% phosphoric acid. Alternatively, heating compounds of formula VIII-b wherein W¹ is -COO(C₁-C₂ alkyl) or -CONH₂ with a compound of the formula R³CONH₂ at a temperature from about 180°C to about 230°C will afford a compound of formula IV-d. Compounds of formula V-c may be prepared, as shown in Scheme 3, by heating compounds of the formula VIII-b wherein W¹ is CN with an excess of a compound having the formula R³CONH₂, at about the reflux temperature.

Compounds of formula I-A, wherein X is O, S, or NR⁸ may be prepared as illustrated in Scheme 4, starting with compounds of formula IX. Compounds of formula X, wherein R⁴ is H and X is O may be prepared by reducing the corresponding compounds of formula IX using, for example, LiAlH₄ or diisobutylaluminum hydride in THF, ethyl ether or dioxane, at temperature from about room temperature to about the reflux temperature. Compounds of the formula X wherein R⁴ is hydrogen and X is sulfur may be prepared by standard methods known in literature for the conversion of - CH₂OH groups to the corresponding -CH₂SH groups. Oxidation of compounds of formula X wherein R⁴ is H and X is O with PCC (pyridinium chlorochromate) using methods described in the literature will provide the corresponding compounds containing a formyl group. Grignard addition (using a Grignard reagent of the formula R⁴MgBr) to such formyl group will afford a compound of formula X wherein R⁴ is as defined previously for formula I. Reductive amination of such formyl group using standard literature methods will provide compounds of the formula X wherein R⁴ is H and X is N. Alternatively, conversion of the carboxylic acid of compounds of the formula IX into the corresponding -CONR⁶ groups, followed by reduction using BH₃•DMS or LiAlH₄ will afford compounds of formula X wherein R⁴ is H and X is NR⁸.

Compounds of formulas I-A and I-C may be prepared from compounds of the formulas X and IX, respectively, as illustrated in Scheme 4, by reacting compounds of formula X wherein X is S, NR⁸, or 0 with a compound of the formula R⁶CHO or R⁶CH(OC₁-C₂ alkyl)₂ and an acid catalyst (such as p-TsOH, HCl, HBr, H₂SO₄ or HCl) in an inert solvent such as toluene, xylene or benzene, preferably toluene, with from none to ten equivalents of water, at temperature from about 70°C to about 160°C, under a Dean-Stark trap apparatus or in the presence of anhydrous sodium sulfate. Compounds of formula I-B and I-D may be prepared by reacting of compounds of formula X and IX, respectively, with triphosgene or thiophosgene and a base such as triethylamine or pyridine in an inert organic solvent such as methylene chloride, THF, dioxane, ether, benzene, chloroform, preferably methylene chloride or dry THF, at temperature from about 0°C to about 25°C.

Compounds of formula I-G, I-E, I-Q and I-F may be prepared as shown in Scheme 5, starting with compounds of formula X wherein X is OH. Compounds of formula XI may be prepared by reacting compounds of formula X with an excess of thionyl chloride in anhydrous methylene chloride at about room temperature. The solvent and excess of thionyl chloride is then removed and the residue is reacted with compound having the formula of Na- , K- or Li-CR⁴(COOC₁-C₄ alkyl)₂ or Na-, K- or Li-CR⁴(CN), in an appropriate solvent such as DMSO, THF, NMP, sulfolane, or a C₁-C₄ alkanol, at a temperature from about room temperature to about 100°C, preferably at about room temperature. Compounds of formula I-Q may be prepared using standard amide cyclization methods known in literature. Such methods include acid cyclization (such as heating in 40-85% phosphoric acid at a temperature from about 100°C to about 150°C; heating in aqueous acetic acid/HCl, or base hydrolysis, decarboxylation, followed by amide cyclization). Compounds of formula I-E may be prepared by bromination of compounds of formula I-Q, followed by base (such DBU or DBN) elimination. Compounds of formula I-F and I-G may be obtained by reducing compounds of the formula of I-Q and I-E, respectively, by standard reduction methods such as heating with BH₃•DMS or BH₃ in THF, or with LiAlH₄ in THF.

Compounds of formula I-H to I-L wherein (R)ₙ represents from zero to three substituents such as R⁴, R⁶, R⁸, R⁹ or R¹² may be prepared starting with compounds of formula XII wherein X is NR⁸, O, or S, as illustrated in Scheme 6. Compounds of the formula XIII may be prepared by reacting the corresponding compounds of formula XII with an acyl halide (such as X¹CH(R⁶)COL (X¹ is chloro, bromo, iodo, mesylate or tosylate, and L is chloro, bromo or iodo)) in the presence of a base such as a tri-(C₁-C₄ alkyl)amine, pyridine or a substituted pyridine, in an appropriate solvent such as methylene chloride, chloroform, THF, DMSO, dioxane, ether or dimethoxyethane (DME), at temperatures from about 0°C to about 180°C, preferably from about room temperature to about 60°C. Compounds of formula I-H may be prepared by reacting compounds of the formula XIII with a base. Suitable bases for use in this reaction include sodium, sodium hydride, potassium hydride, lithium diisopropylamide, butyl lithium, lithium bis(trimethylsilyl)amide, sodium diisopropylamide and sodium or potassium carbonate. Alkylation of compounds having the formula I-H with a base, followed by quenching with an alkyl halide in an appropriate solvent such as ether, THF, methylene chloride, dioxane, benzene, toluene or DME, with or without HMPA, at a temperature from about -78°C to about room temperature, will afford compounds of the formula I-J. Suitable bases for this reaction include lithium diisopropylamide, lithium bis(trimethylsilyl)amide sodium diisopropylamide and butyl lithium. Reaction of compounds having the formula I-H or I-J with a reducing agent such as BH₃•DMS, BH₃, diisbutylaluminum hydride or lithium aluminum hydride will afford compounds of the formula I-K or I-I, respectively. Reaction of compounds of formula I-H or I-J with POCl₃ or PCl₅, followed by reaction with an organometallic agent containing an R⁶ group (such as R⁶₃Al or R⁶₂Zn) will yield compounds of the formula I-I or I-K with an additional R⁶ substituent at the atom next to the N-R⁵ moiety.

Compounds of formula I-M to I-P may be prepared , as illustrated in Scheme 7, by methods analogous to those described in Scheme 6. Double bond formation as shown in formulas I-N, I-O, and I-P may be achieved by bromination followed by elimination, using standard methods known in literature. Alternatively, compounds of formula I-N, I-O, and I-P can be prepared by reacting compounds of formula I-M with a base, and the quenching with PhSeSePh, PhSSO₂Ph, PhSSOPh, PhSSPh or an equivalent agent, followed by oxidation with NalO₄ and elimination with a base. Monocyclic pyridine or pyrimidine starting agents, such compounds of the formulas IX, X and XIV may be prepared by methods analogous to those described in PCT Patent Application PCT/IB95/00373, which designates the United States and was filed on May 18, 1995 and published on December 21, 1995.

The acid addition salts of compounds of the formula can be prepared in a conventional manner by treating a solution or suspension of the corresponding free base with one chemical equivalent of a pharmaceutically acceptable acid. Conventional concentration or crystallization techniques can be employed to isolate the salts. Illustrative of suitable acids are acetic, lactic, succinic, maleic, tartaric, citric, gluconic, ascorbic, benzoic, cinnamic, fumaric, sulfuric, phosphoric, hydrochloric, hydrobromic, hydroiodic, sulfamic, sulfonic acids such as methanesulfonic, benzene sulfonic, p-toluenesulfonic, and related acids.

The compounds of formula I and their pharmaceutically acceptable salts (hereinafter referred to, collectively, as "the active compounds of this invention") may be administered alone or in combination with pharmaceutically acceptable carriers, in either single or multiple doses. Suitable pharmaceutical carriers include inert solid diluents or fillers, sterile aqueous solutions, oils (e.g., peanut oil, sesame oil) and various organic solvents. The pharmaceutical compositions formed by combining the novel compounds of formula I and pharmaceutically acceptable carriers can then be readily administered in a variety of dosage forms such as tablets, powders, lozenges, emulsions, oil soft gels, syrups, injectable solutions and the like. These pharmaceutical compositions can, if desired, contain additional ingredients such as flavorings, binders, excipients and the like. Thus, for purposes of oral administration, tablets containing various excipients such as sodium citrate, calcium carbonate and calcium phosphate may be employed along with various disintegrants such as starch, methylcellulose, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions containing an active compound of this invention or a pharmaceutically acceptable salt thereof in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. The sterile aqueous media employed are all readily available by standard techniques known to those skilled in the art.

The effective dosages for the active compounds of this invention will depend on the intended route of administration and factors such as the age and weight of the patient, as generally known to a physician. The dosages will also depend on the particular illness to be treated. For instance, the daily dosage for stress-induced illnesses, inflammatory disorders, Alzheimer's disease, gastro-intestinal diseases, anorexia nervosa, hemorrhagic stress and drug and alcohol withdrawal symptoms will generally range from about 0.1 to about 50 mg/kg body weight of the patient to be treated.

Methods that may be used to determine the CRF antagonist activity of the active compounds of this invention and their pharmaceutically acceptable salts are described in Endocrinology, 116, 1653-1659 (1985) and Peptides, 10, 179-188 (1985). The binding activities for compounds of the formula I, expressed as IC₅₀ values, generally range from about 0.5 nanomolar to about 10 micromolar.

Methods that can be used to determine the CRF binding protein inhibiting activity of compounds of the formula I are described in Brain Research, (1997), 745(1,2), 248-256.

The present invention is illustrated by the following examples. It will be understood, however, that the invention is not limited to the specific details of these examples. Melting points are uncorrected. Proton nuclear magnetic resonance spectra (¹H NMR) and C¹³ nuclear magnetic resonance spectra (C¹³ NMR) were measured for solutions in deuterochloroform (CDCl₃) and peak positions are expressed in parts per million (ppm) downfield from tetramethylsilane (TMS). The peak shapes are denoted as follows: s, singlet; d, doublet; t, triplet; q, quartet; m, multiplet; b, broad.

The following abbreviations are used in the Examples: Ph=phenyl; iPr=isopropyl; HRMS=high resolution mass spectrum.

### EXAMPLE 1

### 4-(Butyl-ethyl-amino)-2,6-dimethyl-8-(2,4,6-trimethyl-phenyl)-5,8-dihydro-6H-pyrido[2,3-d]pyrimidin-7-one

A mixture of 4-chloro-2,6-dimethyl-8-(2,4,6-trimethyl-phenyl)-5,8-dihydro-6H-pyrido[2,3-d]pyrimidin-7-one (75 mg, 0.227 mmol) and N-butyl-ethyl-amine (65 mg, 0.682 mmol) in DMSO (1 ml) was heated in an oil bath of 135°C for 15 hours. The reaction mixture was quenched with water and extracted with ethyl acetate. The organic layer was dried and concentrated to give 114 mg of the crude material. Silica gel column purification using 5% ethyl acetate in hexane as eluent provided 50 mg of the title compound as a colorless oil. ¹H NMR (CDCl₃) δ 6.95 (s,1H), 6.94(s,1H), 3.2-3.55(m,4H), 2.88-3.05(dd,1H), 2.70-2.85(m,1H), 2.55-2.70(m,1H), 2.35(s,3H), 2.25(s,3H), 2.05(s,3H),1.97(s,3H), 1.5-1.65(m,2H), 1.3-1.5(m,2H), 1.35(d,3H), 1.2(t,3H), 0.98(t,3H) ppm.

### EXAMPLE 2

### 8-(1-Ethyl-propoxy)-6-methyl-4-(2,4,6-trimethyl-phenyl)-3,4-dihydro-1H-pyrido[2,3-b]pyrazin-2-one

To a cooled solution of 2-chloro-N-[4-(1-ethyl-propoxy)-6-methyl-2-(2,4,6-trimethyl-phenylamino)-pyridin-3-yl]-acetamide (40 mg, 0.099 mmol) in dry THF was added 1.0 M lithium bistrimethylsilyl amide (LiN(SiMe₃)₂) in THF (0.3 ml, 0.3 mmol) at -78°C and stirred at that temperature for 1 hour, then warmed to room temperature for 30 min. The mixture was quenched with water and extracted with ethyl acetate. The organic layer was dried and concentrated to give 38 mg of the title compound as a tan crystals. The crystals were purified through silica gel column chromatography using 5% ethyl acetate in hexane as eluent to give 29 mg (81%) of the title compound as a white crystal, mp 179-181°C. ¹H NMR (CDCl₃) δ 7.75(s,1H), 6.95(s,2H), 6.09(s,1H), 4.22(s,2H), 4.22(m,1H), 2.32(s,3H), 2.17(s,3H), 2.16(s,6H), 1.71(m,4H), 0.97(m,6H)ppm.

### EXAMPLE 3

### 8-(1-Ethyl-propoxy)-6-methyl-4-(2,4,6-trimethyl-phenyl)-1,2,3,4-tetrahydro-pyrido[2,3-b]pyrazine

A mixture of 8-(1-ethyl-propoxy)-6-methyl-4-(2,4,6-trimethyl-phenyl)-3,4-dihydro-1H-pyrido[2,3-b]pyrazin-2-one (13 mg, 0.0354 mmol) and 2M borane dimethylsulfide complex (BH₃•DMS) (0.044 ml, 0.0884 mmol) in 2 ml of dry THF was heated at reflux for 2 hours. The mixture was quenched with 0.2 ml of methanol and 0.2 ml of concentrated hydrochloric acid (HCl) and the resulting mixture was stirred at room temperature for 2 hours, and then concentrated to dryness. The residue was quenched with water, neutralized with saturated sodium bicarbonate and extracted with ethyl acetate. The organic layer was washed with brine, dried and concentrated to give 14.7 mg of the title compound as a brown crystals. The crystals were purified through silica gel column chromatography using 10% ethyl acetate in hexane as eluent to give 9 mg of the title compound as a colorless oil. ¹H NMR (CDCl₃) δ 6.93(s,2H), 6.02(s,1H), 4.18(m,1H), 3.62(m,2H), 3.44(m,2H), 2.31 (s,3H), 2.12(s,9H), 1.71(m,4H), 0.98(t,6H)ppm.

### EXAMPLE 4

### 8-(1-Ethyl-propoxy)-1,6-dimethyl-4-(2,4,6-trimethyl-phenyl)-3,4-dihydro-1H-pyrido[2,3-b]pyrazin-2-one

To a -78°C solution of 8-(1-ethyl-propoxy)-6-methyl-4-(2,4,6-trimethyl-phenyl)-3,4-dihydro-1H-pyrido[2,3-b]pyrazin-2-one (50 mg, 0.136 mmol) in 3 ml of dry THF was added 1.0M of LiN[Si(CH₃)₃]₂ in THF (0.14 ml, 0.14 mmol) at -78°C. After stirring at that temperature for 20 min, the reaction mixture was warmed to room temperature and stirred at room temperature overnight. The mixture was quenched with water and saturated ammonium chloride and extracted with ethyl acetate. The organic extract was washed with brine, dried and concentrated to give 51 mg of a golden oil. The oil was purified through silica gel column chromatography using 10% ethyl acetate in hexane as eluent to give 41 mg (79%) of the title compound as a golden oil. ¹H NMR (CDCl₃) δ 6.9(s,2H), 6.17(s,1H), 4.30(m,1H), 4.01(s,2H), 3.47(s,3H), 2.30(s,3H), 2.20(s,3H), 2.01(s,6H), 1.70(m,4H), 0.97(t,6H)ppm.

### EXAMPLE 5

### 4-(1-Ethyl-propoxy)-2-methyl-8-(2,4,6-trimethyl-phenyl)-quinoline

To a solution of 3-pentanol (5.8 ml, 52.7 mmol) in dry THF (5 ml) was added sodium hydride (NaH) portionwise over a period of 10 min. A solution of 4-chloro-2-methyl-8-(2,4,6-trimethyl-phenyl)-quinoline (4.0006 g, 13.52 mmol) in dry THF (10 ml) was added. After stirring at room temperature as for 10 min, 15 ml of dry DMSO was added. The resulting mixture was heated in a 12°C oil bath for 1.5 hours. The mixture was quenched with water and extracted with EtOAc. The organic layer was separated, dried, filtered, and concentrated to give the title compound as 5.002 g of a yellow solid. ¹H NMR (CDCl₃) δ 8.19(d,1H), 7.42(m,2H), 6.96(s,2H), 6.53(s,1H), 4.41(m,1H), 2.51 (s,3H), 2.36(s,3H), 1.89(s,6H), 1.84(m,4H), 1.02(t,6H)ppm.

The yellow solid was prepared as the corresponding HCl salt and concentrated to dryness. The residue was triturated with hexane to give off-white solid. The solid was recrystallized fron EtOAc to give 4.020 g (78%) of white crystals, mp 153-156°C. ¹H NMR (CDCl₃) δ 14.05(brs,1H), 8.33(dd,1H), 7.74(m,1H), 7.66(m,1H), 7.08(s,2H), 6.97(s,1H), 4.76(m,1H), 3.13(s,3H), 2.06(s,3H), 1.8-2.0(m,4H), 1.91 (s,6H), 1.06(t,6H)ppm.

### EXAMPLE 6

### 5-(1-Ethyl-propoxy)-7-methyl-1-(2,4,6-trimethyl-phenyl)-1,4-dihydro-2H-3-oxa-1,8-diaza-naphthalene

A mixture of [4-(1-ethyl-propoxy)-6-methyl-2-(2,4,6-trimethylphenylamino)-pyridin-3-yl]-methanol (79 mg, 0.231 mmol) , 37% aqueous formaldehyde (0.1 ml) and p-TsOH (22 mg, 0.116 mmol) in 10 ml of toluene was heated at reflux using a Dean-Stark apparatus for 3 hours. The mixture was quenched with water and extracted with ethyl acetate. The organic layer was separated, dried and concentrated to give 100 mg of the crude material. The crude material was purified through silica gel column chromatography using 2% methanol in chloroform as eluent to give 40 mg (50%) of the title compound as a clear oil. ¹H NMR (CDCl₃) δ 6.90(s,2H), 6.04(s,1H), 4.87(2 sets of s, 4H), 4.16(m,1H), 2.28(s,3H), 2.19(s,3H), 2.14(s,6H), 1.67(m,4H), 0.94(t,6H) ppm.

### EXAMPLE 7

### 5-(1-Ethyl-propoxy)-7-methyl-1-(2,4,6-trimethyl-phenyl)-1,2-dihydro-3-oxa-1,8-diaza-naphthalen-4-one

The title compound was prepared by the method analogous to that described in Example 6 starting from 4-(1-ethyl-propoxy)-6-methyl-2-(2,4,6-trimethyl-phenylamino)-nicotinic acid to give the title compound as an oil. ¹H NMR (CDCl₃) δ 6.92(s,2H), 6.18(s,1H), 5.21(s,2H), 4.30(m,1H), 2.30(s,3H), 2.25(s,3H), 2.12(s,6H), 1.80(m,4H), 1.02(t,6H)ppm.

### EXAMPLE 8

### 8-( 1-Ethyl-propoxy)-1,6-dimethyl-4-(2,4,6-trimethyl-phenyl)-1,2,3,4-tetrahydro-pyrido[2,3-b]pyrazine

A mixture of 8-(1-ethyl-propoxy)-1,6-methyl-4-(2,4,6-trimethyl-phenyl)-3,4-dihydro-1H-pyrido[2,3-b]pyrazin-2-one (50 mg, 0.131 mmol) and 2M BH₃•DMS (0.16 ml, 0.32 mmol) in 3 ml of dry THF was heated at reflux for 3 hours. The mixture was quenched with 0.5 ml of 1N HCl and the resulting mixture was stirred at room temperature for 20 min, concentrated to dryness. The residue was quenched with water, neutralized with saturated sodium bicarbonate and extracted with ethyl acetate. The organic layer was washed with brine, dried and concentrated to give 38 mg of the title compound as a brown crystals. The crystals was purified through silica gel column chromatography using 10% ethyl acetate in hexane as eluent to give 22 mg of the title compound as a colorless oil. ¹H NMR (CDCl₃) δ 6.91(s,2H), 6.01(s,1H), 4.19(m,1H), 3.44(m,2H), 3.16(m,2H), 2.77 (s,3H), 2.29(s,3H), 2.12(s,3H), 2.07(s,6H), 1.75(m,4H), 0.99(t,6H)ppm.

### EXAMPLE 9

### (1-Ethyl-propyl)-[2-methyl-8-(2,4,6-trimethyl-phenyl)-quinolin-4-yl]-amine

Amixture of 4-bromo-2-methyl-8-(2,4,6-trimethyl-phenyl)-quinoline (130mg, 0.365 mmol), 1-ethylpropylamine (0.13 ml, 1.095 mmol), Pd(OAc)₂ (1.7 mg, 0.073 mmol), BINAP(4.55 mg, 0.0073 mmol) and sodium t-butoxide (49 mg, 0.51 mmol) in 2 ml of toluene was heated in 130-150°C oil bath for 5 hours. The mixture was quenched with water and extracted with I-propyl ether. The organic extract was dried and concentrated to give 160 mg of crude material. The crude material was purified through silica gel column chromatography using 5% to 15% methanol in chloroform as eluent to give 78 mg (62%) of the title compound as a light yellow oil. ¹H NMR(CDCl₃) δ 7.80(m,1H), 7.38(m,1H), 7.33(m,1H), 6.96(s,2H), 6.28(s,1H), 3.45(m,1H), 2.42(s,3H), 2.36(s,3H), 1.90(s,6H), 1.6-1.8(m,4H), 1.20(t,6H) ppm. The corresponding HCl salt was prepared as a light yellow solid. ¹H NMR(CDCl₃) δ 9.87(brs,1H), 9.80(s,1H), 9.62(d,1 H), 7.62(t,1H), 7.44(d,1H), 6.33(s,1H), 3.62(m,1H), 2.55(s,3H), 2.37(s,3H), 2.34(s,3H), 2.15(m,4H), 1.87(s,6H), 0.97(t,6H)ppm.

### EXAMPLE 10

### 5-(1-Ethyl-propoxy)-7-methyl-1-(2,4,6-trimethyl-phenyl)-3,4-dihydro-1H-[1,8]naphthyridin-2-one

A mixture of 2-[4-(1-ethyl-propylamino)-6-methyl-2-(2,4,6-trimethyl-phenylamino)-pyridin-3-ylmethyl]-malonic acid dimethyl ester (100 mg, 0.219 mmol), 85% phosphoric acid (3 ml) and water (3 ml) was heated at reflux for 2 hours. The reaction mixture was cooled to room temperature, diluted with water and extracted with ethyl acetate. The organic layer was washed with brine, dried over anhydrous MgSO₄, filtered and concentrated to dryness to give 91 mg of a clear oil. The oil was purified through silca gel column chromatography using 10% methanol (MeOH) in methylene chloride (CHCl₂) as eluent to give a tan crystals, mp 138-140°C. ¹H NMR (CDCl₃) δ 6.93(s,2H), 6.31(s,1H), 4.21(m,1H), 2.93(m,2H), 2.76(m,2H), 2.31(s,3H), 2.19(s,3H), 1.99(s,6H), 1.71(m,4H), 0.96(t,6H) ppm.

### EXAMPLE 11

### 5-(1-Ethyl-propylamino)-7-methyl-1-(2,4,6-trimethyl-phenyl)-3,4-dihydro-1H-[1,8]naphthyridin-2-one

The title compound was prepared as a tan solid, mp 124-126°C, using a method analogous to that described in the Example 10, starting from 2-[4-(1-ethyl-propylamino)-6-methyl-2-(2,4,6-trimethyl-phenylamino)-pyridin-3-ylmethyl]-malonic acid dimethyl ester and aqueous phosphoric acid. ¹H NMR (CDCl₃) δ 6.91(s,2H), 6.09(s,1H), 3.68(d,1H), 3.33(m,1H), 2.82(m,2H), 2.67(m,2H), 2.30(s,3H), 2.12(s,3H), 1.99(s,6H), 1.5-1.7(m,4H), 0.94(t,6H) ppm.

### Example 12

### 5-(1-Ethyl-propoxy)-7-methyl-1-(2,4,6-trimethyl-phenyl)-3,4-dihydro-1H-pyrido[2,3-d]pyrimidin-2-one

To a mixture of 3-aminomethyl-4-N-(1-ethyl-propyl)-6-methyl-2-N-(2,4,6-trimethyl-phenyl)-pyridine-2,4-diamine (100 mg, 0.293 mmol) in dry THF was added triphosgene (34 mg, 0.114 mmol) at 0°C. The reaction mixture was allowed to gradually warm to room temperature and was stirred for 1 hour. The mixture was quenched with water and extracted with ethyl acetate. The organic layer was dried and concentrated to dryness to give 100 mg (92.5%) of a tan solid. The solid was purified through silica gel column chromatography using 20% to 40% EtOAc in hexane as the eluent to give 75 mg (69.4%) of the title compound as a white crystalline solid, mp 258-260°C. ¹H NMR (CDCl₃) δ 6.92(s,2H), 6.24(s,1H), 5.19(s,1H), 4.48(s,2H), 4.20(m,1H), 2.30(s,3H), 2.19(s,3H), 2.07(s,6H), 1.67(m,4H), 0.94(t,6H) ppm.

### EXAMPLE 13

### 4-(1-Ethyl-propoxy)-2,6-dimethyl-8-(2,4,6-trimethyl-phenyl)-8H-pteridin-7-one

To a solution of 6-(1-ethyl-propoxy)-2-methyl-4-N-(2,4,6-trimethyl-phenyl)-pyrimidine-4,5-diamine (100 mg, 0.305 mmol) in 2 ml of ethanol was added pyruvic acid (30 mg, 0.335 mmol) and the resulting mixture was heated at reflux for 1 hour. An additional 60 mg of pyruvic acid was added and the resulting mixture was heated at reflux overnight. The mixture was quenched with water and extracted with chloroform. The organic layer was washed with water and brine, dried over sodium sulfate, filtered and concentrated to give an oil residue. The residue was purified through silica gel column chromatography using hexane to 15% ethyl acetate in hexane as eluent to give the title compound as a yellow solid. ¹H NMR (CDCl₃) δ 6.99 (s,2H), 5.39(m,1H), 2.61(s,3H), 2.40(s,3H), 2.35(s,3H), 1.88(s,6H), 1.7-1.9(m,4H), 0.99(t,6H)ppm.

### EXAMPLE 14

### 5-(1-Ethyl-propoxy)-7-methyl-1-(2,4,6-trimethyl-phenyl)-1,2,3,4-tetrahydro-[1,8]naphthyridine

The title compound was prepared in a 86% yield as a clear oil by the method analogous to that described in Example 8 starting from 5-(1-ethyl-propoxy)-7-methyl-1-(2,4,6-trimethyl-phenyl)-3,4-dihydro-1H-[1,8]naphthyridin-2-one and BH3•DMS in THF.

¹H NMR (CDCl₃) δ 6.90(s,2H), 5.95(s,1H), 4.13(m,1H), 3.40(m,2H), 2.71(m,2H), 2.28(s,3H), 2.14(s,3H), 2.08(s,6H), 1.99(m,2H), 1.67(m,4H), 0.94(t,6H) ppm.

### EXAMPLE 15

### 8-(1-Ethyl-propoxy)-2,6-dimethyl-4-(2,4,6-trimethyl-phenyl)-4H-pyrido[2,3-b]pyrazin-3-one

A mixture of 4-(1-ethyl-propoxy)-6-methyl-N-2-(2,4,6-trimethyl-phenyl)-pyridine- 2,3-diamine (250 mg, 0.763 mmol) and pyruvic acid (67 mg, 0.763 mmol) in 8ml of EtOH was heated at reflux overnight. The reaction mixture was cooled and a pale yellow crystalline precipitate formed and filtered to give 83 mg of the title compound, mp 215-217°C. The filtrate was concentrated to dryness to give an additional 200 mg of the desired product as a yellow solid. ¹H NMR (CDCl₃) δ 6.98(s,2H), 6.53(s,1H), 4.37(m,1H), 2.61(s,3H), 2.34(s,3H), 1.87(s,6H), 1.8-2.0(m,4H), 1.04(t,6H)ppm.

The title compounds of Examples 16 and 17 were isolated starting from 4-(1-ethyl-propoxy)-6-methyl-2-(2,4,6-trimethyl-phenylamino)-nicotinamide and triphosgene, using a method analogous to that described in Example 12.

### EXAMPLE 16

### 4-Chloro-5-(1-ethyl-propoxy)-7-methyl-1-(2,4,6-trimethyl-phenyl)-1H-pyrido[2,3-d]pyrimidin-2-one

A white crystal, mp 125-127°C. ¹H NMR (CDCl₃) δ 6.93(s,3H), 6.56(s,1H), 4.31(m,1H), 2.35(s,3H), 2.34(s,6H), 2.30(s,3H), 1.76(m,4H), 0.97(t,6H) ppm.

### EXAMPLE 17

### 5-(1-Ethyl-propoxy)-7-methyl-1-(2,4,6-trimethyl-phenyl)-1H-pyrido[2,3-d]pyrimidine-2,4-dione,

A white crystal, mp 105-107°C. ¹H NMR (CDCl₃) δ 6.90(s,2H), 6.26(brs,1H), 6.05(s,1H), 4.24(m,1H), 2.29(s,3H), 2.25(s,3H), 2.17(s,6H), 1.71(m,4H), 0.97(t,6H) ppm.

The title compounds of Examples 18 and 19 were prepared starting from [2-(4-bromo(or chloro)-2,6-dimethyl-phenylamino)-4-(1-ethyl-propoxy)-6-methyl-pyridin -3-yl]-methanol and 37% aqueous formaldehyde using a procedure analogous to that described in Example 6.

### EXAMPLE 18

### 1-(4-Bromo-2,6-dimethyl-phenyl)-5-(1-ethyl-propoxy)-7-methyl-1,4-dihydro-2H-3-oxa-1,8-diaza-naphthalene

The parent compound is a clear oil. ¹H NMR (CDCl₃) δ 7.20 (s,2H), 6.05(s,1H), 4.85(s,2H), 4.83(s,2H), 4.14(m,1H), 2.17(s,3H), 2.12(s,6H), 1.65(m,4H), 0.92(t,6H) ppm.

The HCl salt, a white solid, mp 206-209°C. ¹H NMR (CDCl₃) δ 14.5(brs,1H), 7.31(s,2H), 6.23(s,1H), 4.84(s,2H), 4.81(s,2H), 4.34(m,1H), 2.76(s,3H), 2.20(s,6H), 1.72(m,4H), 0.94(t,6H) ppm.

### EXAMPLE 19

### 1-(4-Chloro-2,6-dimethyl-phenyl)-5-(1-ethyl-propoxy)-7-methyl-1,4-dihydro-2H-3-oxa-1,8-diaza-naphthalene

The parent compound is a clear oil. ¹H NMR (CDCl₃) δ 7.07 (s,2H), 6.07(s,1H), 4.87(s,2H), 4.85(s,2H), 4.17(m,1H), 2.19(s,3H), 2.15(s,6H), 1.67(m,4H), 0.95(t,6H)ppm.

The HCl salt, a white solid, mp 190-192°C. ¹H NMR (CDCl₃) d 14.5(brs,1H), 7.26(s,2H), 6.27(s,1H), 4.87(s,2H), 4.85(s,2H), 4.37(m,1H), 2.78(s,3H), 2.23(s,6H), 1.74(m,4H), 0.97(t,6H) ppm.

### PREPARATION A

### 2-Chloro-N-[4-(1-ethyl-propoxy)-6-methyl-2-(2,4,6-trimethyl-phenylamino)-pyridin-3-yl]-acetamide

To a solution of 4-(1-ethyl-propoxy)-6-methyl-N2-(2,4,6-trimethyl-phenyl)-pyridine-2,3-diamine (103 mg, 0.315 mmol) in 4 ml of dry THF was added chloroacetyl chloride (36 mg, 0.315 mmol) and triethylamine (32 mg, 0.315 mmol) at 0°C. The mixture was warmed to room temperature and stirred at room temperature overnight. The mixture was quenched with water and extracted with ethyl acetate. The organic layer was dried and concentrated to give 125 mg of brown residue. The brown residue was purified through silica gel column chromatography using 10% ethyl acetate in hexane as eluent to give 59 mg of the title compounds as a tan solid, mp 79-82°C. ¹H NMR (CDCl₃) δ 8.15(brs,1H), 6.87(s,2H), 6.78(s,1H), 6.14(s,1H), 4.20(m,1H), 4.19(s,2H), 2.28(s,3H), 2.24(s,3H), 2.16(s,6H),

### PREPARATION B

### 4-Chloro-2,6-dimethyl-8-(2,4,6-trimethyl-phenyl)-5,8-dihydro-6H-pyrido[2,3-d]pyrimidin-7-one

A mixture of 3-[4-chloro-2-methyl-6-(2,4,6-trimethyl-phenylamino)-pyrimidin-5-yl]-2-methyl-propionic acid ethyl ester (173 mg, 0.46 mmol) and p-TsOH (56 mg) in 10 ml of toluene was heated at reflux using Dean-stark trap apparatus for 9 hours. The mixture was quenched with water and extracted with ethyl acetate. The organic extract was washed with brine, dried and concentrated to give 184 mg of the crude material.
The crude material was purified through silica gel column chromatography using 10% ethyl acetate in hexane as eluent to give 95 mg of the title compound as white crystals, mp 136-139°C, after recrystallization from ethyl ether. ¹H NMR (CDCl₃) δ 6.95(s,1H), 6.94(s,1H), 3.25(dd,1H), 2.8-3.0(m,2H), 2.41(s,3H), 2.32(s,3H), 1.96(s,3H), 1.93(s,3H), 1.37(d,3H)ppm.

### Preparation C

### 2-Methyl-8-(2,4,6-trimethyl-phenyl)-quinolin-4-ol

A mixture of 2',4',6'-trimethyl-biphenyl-2-ylamine (607 mg, 2.88 mmol) and methyl acetylacetone (607 mg, 5.75 mmol) in polyphosphoric acid (3 ml) was heated in 170°C oil bath for 2.5 hours. The mixture was quenched with water and extracted twice with chloroform. The organic layer was washed with brine, dried and concentrated to give the title compound as an oil. The oil was pumped in vacuo, then trituated with a mixture of ether and hexane to give 642 mg (81 %) of the title compound as a beige solid. The solid was recrystallized from ethyl acetate to give a beige solid, mp >250°C.

¹H NMR (CDCl₃) δ 8.31(d,1H), 7.9(brs,1H), 7.40(m,1H), 7.34(m,1H), 7.01 (s,2H), 6.26(s,1H), 2.33(s,3H), 2.26(s,3H), 1.6(s,3H), 1.93(s,3H), 1.37(d,3H)ppm.

### Preparation D

### 4-Chloro-2-methyl-8-(2,4,6-trimethyl-phenyl)-quinoline

A mixture of 2-methyl-8-(2,4,6-trimethyl-phenyl)-quinolin-4-ol (335 mg, 1.21 mmol) and POCl₃(2.5 ml) was heated in 130°C oil bath for 3 hours. The mixture was cooled and poured into ice-water and extracted with ethyl acetate. The organic layer was washed with brine, dried and concentrated to give 350 mg of crude material as a brown oil. The oil residue was purified through silica gel column chromatography using chloroform as eluent to give 316 mg (87%) of the title compound as a yellow oil. ¹H NMR (CDCl₃) δ 8.20(d,1H), 7.60(m,1H), 7.47(d,1H), 7.35(s,1H), 6.97(s,2H), 2.54(s,3H), 2.36(s,3H), 1.86(s,6H)ppm.

### Preparation E

### Trifluoro-methanesulfonic acid 2-methyl-8-(2,4,6-trimethyl-phenyl)-quinolin-4-yl ester

A mixture of 2-methyl-8-(2,4,6-trimethyl-phenyl)-quinolin-4-ol (416 mg, 1.5 mmol), triflic anhydride (508 ml., 1.8 mmol) and triethylamine (182 mg, 1.8 mmol) in 5 ml of methylene chloride was stirred at room temperature for 1 hour. The mixture was quenched with water and extracted with chloroform. The organic layer was washed with brine, dried and concentrated to give 587 mg of the title compound as a brown glass form. The material was used directly for the next reaction. ¹H NMR (CDCl₃) δ 8.02(d,1H), 7.65(t,1H), 7.55(d,1H), 7.24(s,1H), 6.97(s,2H), 2.62(s,3H), 2.37(s,3H), 1.85(s,6H)ppm.

### Preparation F

### 4-Bromo-2-methyl-8-(2,4,6-trimethyl-phenyl)-quinoline

A mixture of trifluoro-methanesulfonic acid 2-methyl-8-(2,4,6-trimethyl-phenyl)-quinolin-4-yl ester (426 mg, 1 mmol) and potassium bromide (KBr) (809 mg, 1.1 mmol) in a mixture of 1 ml of dry DMSO and 3 ml of dry THF was heated in 120°C oil bath for 3 hours. The mixture was quenched with water, extracted with ethyl acetate. The organic layer was dried and concentrated to give 358 mg of the title compound as an off-white solid. ¹H NMR (CDCl₃) δ 8.16 (m,1H), 7.59 (m,1H), 7.56(s,1H), 7.48(m,1H), 6.97(s,1H), 2.53(s,3H), 2.37(s,3H), 1.87(,6H) ppm.

## Claims

1. A compound of the formula 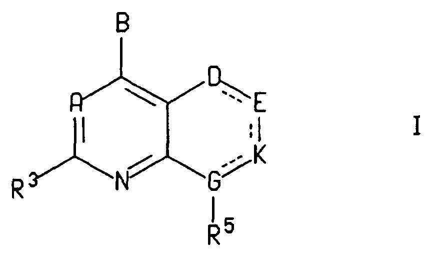
the dashed lines represent optional double bonds;
A is nitrogen or CR⁷;
B is -NR¹R², NHCHR¹R², -OCHR¹R², CHR¹R², or SCHR¹R²;
G is nitrogen or CR⁴ and is single bonded to all atoms to which it is attached, or G is carbon and is double bonded to K;
K is nitrogen or CR⁶ when double bonded to G or E, or K is C=O, CR⁶R¹² or NR⁸ when single bonded to both adjacent ring atoms,
D and E are each, independently, C=O, CR⁴R⁶ or NR⁸ when single bonded to both adjacent ring atoms, or nitrogen or CR⁴ when it is double bonded to an adjacent ring atom;
the 6- or 7-membered ring that contains D, E, K and G may contain from one to three double bonds, from zero to two heteroatoms selected from oxygen, nitrogen and sulfur, and from zero to two C=O or C=S groups, wherein the carbon atoms of such groups are part of the ring and the oxygen and sulfur atoms are substituents on the ring;
R¹ is C₁-C₆ alkyl optionally substituted with from one substituent selected from hydroxy, fluoro, C₁-C₄ alkoxy, CF₃, and cyclopropylfluoro,
wherein the C₁-C₄ alkyl group may optionally contain one or two double or triple bonds;
R² is C₁-C₆ alkyl which may optionally contain from one to three double or triple bonds (C₁-C₂ alkyl)-CO-(C₁-C₂ alkyl) or benzyl, which may optionally be substituted with one substituent selected from chloro, fluoro, hydroxy, C₁-C₂ alkyl, CF_{3,} C₁-C₂ alkoxy, and cyclopropyl (C₁-C₄ alkyl)-CO-(C₁-C₄ alkyl);
-NR¹R² may form a ring selected from saturated 3 to 8 membered rings, the 5 to 8 membered rings of which may optionally contain one or two double bonds, and wherein one or two of the ring carbon atoms of such 5 to 8 membered rings may optionally and independently be replaced by an oxygen or sulfur atom or by NZ² wherein Z² is hydrogen, benzyl or C₁-C₄ alkyl;
R³ is hydrogen, C₁-C₄ alkyl, -O(C₁-C₄ alkyl), chloro, fluoro, bromo, iodo, -S(C₁-C₄ alkyl) or -SO₂(C₁-C₄ alkyl);
each R⁸, and R¹² is selected, independently, from hydrogen and C₁-C₂ alkyl;
each R⁴ and R⁶ that is attached to a carbon atom is selected, independently, from hydrogen and C₁-C₆ alkyl, fluoro, chloro, bromo, iodo, hydroxy, hydroxy (C₁-C₂ alkyl), trifluoromethyl, cyano, amino, nitro, -O(C₁-C₄ alkyl), -N(C₁-C₄ alkyl)(C₁-C₂ alkyl), -CH₂SCH₃, - S(C₁-C₄ alkyl), -CO(C₁-C₄ alkyl), -C(=O)H or -C(=O)O(C₁-C₄ alkyl), wherein each of the C₁-C₂ alkyl moieties in the foregoing R⁴ and R⁶ groups may optionally contain one double or triple bond; and R⁶, when attached to a nitrogen atom, is selected from hydrogen and C₁-C₄ alkyl;
R⁵ is substituted phenyl, pyridyl or pyrimidyl, wherein each of the foregoing R⁵ groups is substituted with two or three substituents R¹³, wherein up to three of said substituents may be selected, independently, from chloro, C₁-C₄ alkyl, -O(C₁-C₄ alkyl), -(C₁-C₄ alkylene)O(C₁-C₆alkyl), may be selected, independently, from fluoro, chloro, bromo, iodo, formyl, cyano, trifluoromethyl, OCF₃, and -(C₁-C₄alkylene)-OH, and wherein the C₁-C₄ alkyl may optionally contain one double or triple bond; R⁷ is hydrogen, methyl, halo (e.g., chloro, fluoro, iodo or bromo), hydroxy, methoxy, -C(=O)(C₁-C₂ alkyl), -C(=O)O(C₁-C₂ alkyl), hydroxymethyl, trifluoromethyl or formyl;
R¹⁰ is hydrogen, hydroxy, methoxy or fluoro; and
R¹¹ is hydrogen or C₁-C₄ alkyl;
with the proviso that in the ring containing D, E, K and G of formula I, there can not be two double bonds adjacent to each other;
and the pharmaceutically acceptable salt of such compound.

2. A compound according to claim 1 wherein A is N, CH or CH₃.

3. A compound according to claim 1 wherein G is nitrogen.

4. A compound according to claim 1 wherein G is carbon and the ring containing D, E, K and G is a benzo ring.

5. A compound according to claim 1 wherein G is N; D is NH, NCH₃; and E---K is CH₂-CH₂, CH=CH, C(O)-CH₂, or CH₂-C(O).

6. A Compound according to claim 1 wherein G is N; D---E---K is C(O)-O-CH₂, CH₂-O-CH₂, C(O)-CH₂-CH₂, C(O)-CH=CH, CH₂-CH₂-CH₂-, CH₂-CH₂-C(O), CH=CH-C(O), CH=CH-CH₂, CH=CH-NH, CH=CH-NCH₃.

7. A compound according to claim 1 wherein R³ is methyl and each of R⁴, R⁶, R⁸, R⁹ and R¹² is hydrogen.

8. A compound according to claim 1 wherein R⁵ is di- or tri-substituted phenyl in which the two or three substitutents are independently selected from C₁-C₄ alkyl, -O-(C₁-C₄ alkyl), -(C₁-C₄ alkylene)-O-(C₁-C₄ alkyl), CF₃, -OCF₃, -CHO, -(C₁-C₄ alkylene)-OH, cyano, chloro, fluoro, bromo and iodo, wherein each of the forgoing (C₁-C₄) alkyl groups may optionally contain one double or triple bond.

9. A compound according to claim 1 wherein R³ is methyl, ethyl, chloro or methoxy and each R⁴, R⁶, R⁸, R⁹, and R¹² is, independently, hydrogen or methyl.

10. A compound according to claim 1 wherein B is -CHR¹R², -NCHR¹R² or -OCHR¹R², and the CHR¹R² group of B is a cyclopentane ring, a tetrahydrofuran ring or a tetrahydrothienyl ring.

11. A pharmaceutical composition for the treatment, prevention or inhibition of (a) a disorder the treatment of which can be effected or facilitated by antagonizing CRF, including but not limited to disorders induced or facilitated by CRF, or (b) a disorder selected from inflammatory disorders such as rheumatoid arthritis and osteoarthritis, pain, asthma, psoriasis and allergies; generalized anxiety disorder; panic;
phobias; obsessive-compulsive disorder; post-traumatic stress disorder; sleep disorders induced by stress; pain perception such as fibromyalgia; mood disorders such as depression, including major depression, single episode depression, recurrent depression, child abuse induced depression, mood disorders associated with premenstrual syndrome, and postpartum depression; dysthemia; bipolar disorders; cyclothymia; chronic fatigue syndrome; stress-induced headache; cancer; irritable bowel syndrome, Crohn's disease; spastic colon; post operative ileus; ulcer;diarrhea; stress-induced fever; human immunodeficiency virus (HIV) infections; neurodegenerative diseases such as Alzheimer's disease, Parkinson's disease and Huntington's disease; gastrointestinal diseases; eating disorders such as anorexia and bulimia nervosa; hemorrhagic stress; chemical dependencies and addictions (e.g., dependencies on alcohol, cocaine, heroin, benzodiazepines, or other drugs); drug and alcohol withdrawal symptoms; stress-induced psychotic episodes; euthyroid sick syndrome; syndrome of inappropriate antidiarrhetic hormone (ADH); obesity; infertility; head traumas; spinal cord trauma; ischemic neuronal damage (e.g., cerebral ischemia such as cerebral hippocampal ischemia); excitotoxic neuronal damage; epilepsy; stroke; immune dysfunctions including stress induced immune dysfunctions (e.g., porcine stress syndrome, bovine shipping fever, equine paroxysmal fibrillation, and dysfunctions induced by confinement in chickens, sheering stress in sheep or human-animal interaction related stress in dogs); muscular spasms; urinary incontinence; senile dementia of the Alzheimer's type; multiinfarct dementia; amyotrophic lateral sclerosis; hypertension; tachycardia; congestive heart failure; osteoporosis; premature birth; and hypoglycemia in a mammal, comprising an amount of a compound according to claim 1 that is effective in the treatment of such disorder, and a pharmaceutically acceptable carrier.

12. Use of a compound as claimed in claim 1 for the preparation of a medicament for the treatment of a disorder as claimed in claim 11.

13. Use of a compound according to claim 1 in the preparation of a medicament for the treatment or prevention of a disorder or condition, the treatment or prevention of which can be effected or facilitated by inhibiting CRH binding protein in a mammal, including a human, comprising administering to said mammal a CRH binding protein inhibiting amount of a compound according to claim 1.

14. A pharmaceutical composition for treating or preventing a disorder or condition, the treatment or prevention of which can be effected or facilitated by inhibiting CRH binding protein in a mammal, including a human, comprising a CRH binding protein inhibiting amount of a compound according to claim 1 and a pharmaceutically acceptable carrier.

15. A compound according to claim 1, wherein G is carbon and the ring containing D, E, K, and G is a benzo ring.

16. A compound according to claim 15, wherein R³ is methyl.

17. A compound according to claim 16, wherein R⁵ is di- or tri-substituted phenyl at the ortho or para positions in which the two or three substituents are independently selected from C₁-C₄ alkyl, cyclopropyl, -O-(C₁-C₄ alkyl), -(C₁-C₄ alkylene)-O-(C₁-C₄ alkyl), CF₃, OCF₃, CHO, -(C₁-C₄ alkylene)-OH, chloro, fluoro, bromo and iodo, wherein each of the foregoing C₁-C₄ alkyl groups may optionally contain one double or triple bond.

18. A compound according to claim 16, wherein R⁵ is di- or tri-substituted pyridyl at the ortho or para positions in which the two or three substituents are independently selected from C₁-C₄ alkyl, cyclopropyl , -O-(C₁-C₄ alkyl), -(C₁-C₄ alkylene)-O-(C₁-C₄ alkyl), CF₃, OCF₃, CHO, -(C₁-C₄ alkylene)-OH, chloro, fluoro, bromo and iodo, wherein each of the foregoing C₁-C₄ alkyl groups may optionally contain one double or triple bond.

19. A compound according to claim 1, wherein G is N; D--E--K is C(C_{zero}-C₁alkyl)-O-C(C_{zero}-C₁ alkyl)(C_{zero}-C₁ alkyl).

20. A compound according to claim 1, wherein G is N; D--E--K is CH₂-O-CH₂.

21. A compound according to claim 20, wherein R³ is methyl.

22. A compound according to claim 21, wherein R⁵ is di- or tri-substituted phenyl at the ortho or para positions in which the two or three substituents are independently selected from C₁-C₄ alkyl, cyclopropyl, -O-(C₁-C₄ alkyl), -(C₁-C₄ alkylene)-O-(C₁-C₄ alkyl), CF₃, OCF₃, CHO, -(C₁-C₄ alkylene)-OH, chloro, fluoro, bromo and iodo, wherein each of the foregoing C₁-C₄ alkyl groups may optionally contain one double or triple bond.

23. A compound according to claim 21, wherein R⁵ is di- or tri-substituted pyridyl at the ortho or para positions in which the two or three substituents are independently selected from C₁-C₄ alkyl, cyclopropyl , -O-(C₁-C₄ alkyl), -(C₁-C₄ alkylene)-O-(C₁-C₄ alkyl), CF₃, OCF₃, CHO, -(C₁-C₄ alkylene)-OH, chloro, fluoro, bromo and iodo, wherein each of the foregoing C₁-C₄ alkyl groups may optionally contain one double or triple bond.

24. A compound according to claim 1, wherein G is N; D--E--K is O-C(C_{zero}-C₁ alkyl)(C_{zero}-C₁ alkyl)- C(C_{zero}-C₁ alkyl)(C_{zero}-C₁ alkyl), S-C(C_{zero}-C₁ alkyl)(C_{zero}-C₁ alkyl)-C(C_{zero}-C₁ alkyl)(C_{zero}-C₁ alkyl) or N(C_{zero}-C₁ alkyl)-C(C_{zero}-C₁ alkyl)(C_{zero}-C₁ alkyl)- C(C_{zero}-C₁ alkyl)(C_{zero}-C₁ alkyl).

25. A compound according to claim 1, wherein G is N; D--E--K is O-CH₂-CH_{2,} O-CH=CH, S-CH₂-CH_{2,} S-CH=CH.

26. A compound according to claim 25, wherein R³ is methyl.

27. A compound according to claim 26, wherein R⁵ is di- or tri-substituted phenyl at the ortho or para positions in which the two or three substituents are independently selected from C₁-C₄ alkyl, cyclopropyl , -O-(C₁-C₄ alkyl), -(C₁-C₄ alkylene)-O-(C₁-C₄ alkyl), CF₃, OCF₃, CHO, -(C₁-C₄ alkylene)-OH, chloro, fluoro, bromo and iodo, wherein each of the foregoing C₁-C₄ alkyl groups may optionally contain one double or triple bond.

28. A compound according to claim 26, wherein R⁵ is di-substituted pyridyl at the ortho or para positions in which the two or three substituents are independently selected from C₁-C₄ alkyl, cyclopropyl , -O-(C₁-C₄ alkyl), -(C₁-C₄ alkylene)-O-(C₁-C₄ alkyl), CF₃, OCF₃, CHO, - (C₁-C₄ alkylene)-OH, chloro, fluoro, bromo and iodo, wherein each of the foregoing C₁-C₄ alkyl groups may optionally contain one double or triple bond.

29. A compound according to claim 1, wherein G is N; D--E--K is NH-CH₂-CH₂, NMe-CH₂-CH₂-N-R⁵, NH-CH=CH-N-R⁵, or NCH₃-CH=CH-N-R⁵.

30. A compound according to claim 29, wherein R³ is methyl.

31. A compound according to claim 30, wherein R⁵ is di- or tri-substituted phenyl at the ortho or para positions in which the two or three substituents are independently selected from C₁-C₄ alkyl, cyclopropyl , -O-(C₁-C₄ alkyl), -(C₁-C₄ alkylene)-O-(C₁-C₄ alkyl), CF₃, OCF₃, CHO, -(C₁-C₄ alkylene)-OH, chloro, fluoro, bromo and iodo, wherein each of the foregoing C₁-C₄ alkyl groups may optionally contain one double or triple bond.

32. A compound according to claim 30, wherein R⁵ is di-substituted pyridyl at the ortho or para positions in which the two or three substituents are independently selected from C₁-C₄ alkyl, cyclopropyl , -O-(C₁-C₄ alkyl), -(C₁-C₄ alkylene)-O-(C₁-C₄ alkyl), CF₃, OCF₃, CHO, - (C₁-C₄ alkylene)-OH, chloro, fluoro, bromo and iodo, wherein each of the foregoing C₁-C₄ alkyl groups may optionally contain one double or triple bond.

33. A compound according to claim 1, wherein G is N; D--E--K is N=C(C_{zero}-C₁ alkyl)-C(=O), N(C_{zero}-C₁ alkyl)-C(=O)-C(C_{zero}-C₁ alkyl), C(=O)-N(C_{zero}-C₁ alkyl)-C(=O), C(C₁)=N-C(=O), C(C_{zero}-C₁ alkyl)=N-C(=O), CH₂CH₂CH₂, CH₂-CH₂-C(=O), CH₂-N(C_{zero}-C₁ alkyl)-C(=O).

34. A compound according to claim 33, wherein R³ is methyl.

35. A compound according to claim 34, wherein R⁵ is di- or tri-substituted phenyl at the ortho or para positions in which the two or three substituents are independently selected from C₁-C₄ alkyl, cyclopropyl, -O-(C₁-C₄ alkyl), -(C₁-C₄ alkylene)-O-(C₁-C₄ alkyl), CF₃, OCF₃, CHO, -(C₁-C₄ alkylene)-OH, chloro, fluoro, bromo and iodo, wherein each of the foergoing C₁-C₄ alkyl groups may optionally contain one double or triple bond.

36. A compound according to claim 34, wherein R⁵ is di-substituted pyridyl at ortho or para positions in which the two or three substituents are independently selected from C₁-C₄ alkyl, cyclopropyl, -O-(C₁-C₄ alkyl), -(C₁-C₄ alkylene)-O-(C₁-C₄ alkyl), CF₃, OCF₃, CHO, -(C₁-C₄ alkylene)-OH, chloro, fluoro, bromo and iodo, wherein each of the foregoing C₁-C₄ alkyl groups may optionally contain one double or triple bond.

37. A compound according to claim 1, wherein said compound is:
4-(Butyl-ethyl-amino)-2,6-dimethyl-8-(2,4,6-trimethyl-phenyl)-5, 8-dihydro-6H-pyrido[2,3-d]pyrimidin-7-one;
8-(1-Ethyl-propoxy)-6-methyl-4-(2,4,6-trimethyl-phenyl)-3, 4-dihydro-1H-pyrido[2,3-b]pyrazin-2-one;
8-(1-Ethyl-propoxy)-6-methyl-4-(2,4,6-trimethyl-phenyl)-1,2,3,4-tetrahydro-pyrido[2,3-b] pyrazine;
8-(1-Ethyl-propoxy)-1,6-dimethyl-4-(2,4,6-trimethyl-phenyl)-3,4-dihydro-1H-pyrido[2,3-b ]pyrazin-2-one;
5-(1-Ethyl-propoxy)-7-methyl-1-(2,4,6-trimethyl-phenyl)-1,4-dihydro-2H-3-oxa-1,8-diaza - naphthalene;
5-(1-Ethyl-propoxy)-7-methyl-1-(2,4,6-trimethyl-phenyl)-1,2-dihydro-3-oxa-1,8-diaza-na phthalen-4-one;
8-(1-Ethyl-propoxy)-1,6-dimethyl-4-(2,4,6-trimethyl-phenyl)-1,2,3,4-tetrahydro-pyrido[2,3-b]pyrazine;
(1-Ethyl-propyl)-[2-methyl-8-(2,4,6-trimethyl-phenyl)-quinolin-4-yl]-amine;
4-(1-Ethyl-propoxy)-2-methyl-8-(2,4,6-trimethyl-phenyl)-quinoline;
2-Methyl-4-(tetrahydro-furan-3-yloxy)-8-(2,4,6-trimethyl-phenyl)-quinoline;
5-(1-Ethyl-propoxy)-7-methyl-1-(2,4,6-trimethyl-phenyl)-3,4-dihydro-1H-[1,8]naphthyridin-2-one;
5-(1-Ethyl-propylamino)-7-methyl-1-(2,4,6-trimethyl-phenyl)-3,4-dihydro-1H-[1,8]naphthyridin-2-one;
5-(1-Ethyl-propoxy)-7-methyl-1-(2,4,6-trimethyl-phenyl)-3,4-dihydro-1H-pyrido-[2,3-d]pyrimidin-2-one;
4-(1-Ethyl-propoxy)-2,6-dimethyl-8-(2,4,6-trimethyl-phenyl)-8H-pteridin-7-one;
5-(1-Ethyl-propoxy)-7-methyl-1-(2,4,6-trimethyl-phenyl)-1,2,3,4-tetrahydro-[1,8]naphthyr idine;
8-(1-Ethyl-propoxy)-2,6-dimethyl-4-(2,4,6-trimethyl-phenyl)-4H-pyrido-[2,3-b]pyrazin-3-one;
4-Chloro-5-(1-ethyl-propoxy)-7-methyl-1-(2,4,6-trimethyl-phenyl)-1H-pyrido-[2,3-d]pyrimidin-2-one;
5-(1-Ethyl-propoxy)-7-methyl-1-(2,4,6-trimethyl-phenyl)-1H-pyrido-[2,3-d]pyrimidine-2,4-dione;
1-(4-Bromo-2,6-dimethyl-phenyl)-5-(1-ethyl-propoxy)-7-methyl-1,4-dihydro-2H-3-oxa-1, 8-diaza-naphthalene;
1-(4-Chloro-2,6-dimethyl-phenyl)-5-(1-ethyl-propoxy)-7-methyl-1,4-dihydro-2H-3-oxa-1, 8-diaza-naphthalene;
or a pharmaceutically acceptable salt of such compound.

38. A compound of the formula 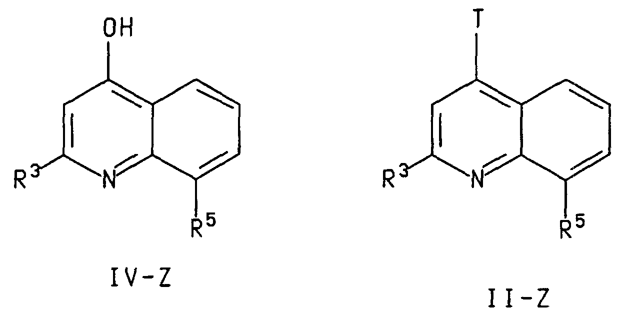 wherein R³, R⁷ and R⁵ are defined as in claim 1 and T is Cl, Br, I or OTf.

## Patentansprüche

1. Verbindung der Formel 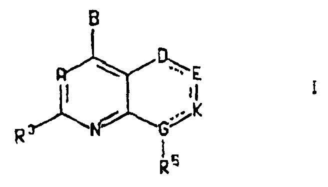 die Strichlinien geben wahlweise Doppelbindungen wieder;
A ist Stickstoff oder CR⁷;
B ist -NR¹R², NHCHR¹R², -OCHR¹R², CHR¹R² oder SCHR¹R²;
G ist Stickstoff oder CR⁴ und ist einfach gebunden an alle Atome, an die es gebunden ist, oder G ist Kohlenstoff und ist doppelt an K gebunden;
K ist Stickstoff oder CR⁶, wenn doppelt an G oder E gebunden, oder K ist C=O, CR⁶R¹² oder NR⁸, wenn einfach an beide benachbarte Ringatome gebunden;
D und E sind jeweils unabhängig C=O, CR⁴R⁶ oder NR⁸, wenn einfach gebunden an beide benachbarte Ringatome, oder Stickstoff oder CR⁴, wenn es doppelt an ein benachbartes Ringatom gebunden ist;
der 6- oder 7-gliedrige Ring, der D, E, K und G enthält, kann ein bis drei Doppelbindungen, null bis zwei Heteroatome, ausgewählt aus Sauerstoff, Stickstoff und Schwefel, und null bis zwei Gruppen C=O oder C=S, wobei die Kohlenstoffatome solcher Gruppen Teil des Rings sind und die Sauerstoff- und Schwefelatome Substituenten an dem Ring sind, enthalten;
R¹ ist C₁-C₆-Alkyl, gegebenenfalls substituiert mit einem Substituenten, ausgewählt aus Hydroxy, Fluor, C₁-C₄-Alkoxy, CF₃ und Cyclopropylfluor;
wobei die C₁-C₄-Alkylgruppe gegebenenfalls eine oder zwei Doppel- oder Dreifachbindungen enthalten kann;
R² stellt C₁-C₆-Alkyl, das gegebenenfalls eine bis drei Doppel- oder Dreifachbindungen enthalten kann, (C₁-C₂-Alkyl)-CO-(C₁-C₂-alkyl) oder Benzyl, gegebenenfalls substituiert mit einem Substituenten, ausgewählt aus Chlor, Fluor, Hydroxy, C₁-C₂-Alkyl, CF₃, C₁-C₂-Alkoxy und Cyclopropyl-(C₁-C₄-alkyl)-CO-(C₁-C₄-alkyl), dar;
-NR¹R² kann einen Ring bilden, ausgewählt aus gesättigten 3- bis 8-gliedrigen Ringen, wobei die 5- bis 8-gliedrigen Ringe davon gegebenenfalls eine oder zwei Doppelbindungen enthalten können, und wobei eines oder zwei der Ringkohlenstoffatome von solchen 5- bis 8-gliedrigen Ringen gegebenenfalls und unabhängig durch ein Sauerstoff- oder Schwefelatom oder durch NZ², worin Z² Wasserstoff, Benzyl oder C₁-C₄-Alkyl darstellt, ersetzt sein können;
R³ stellt Wasserstoff, C₁-C₄-Alkyl, -O(C₁-C₄-Alkyl), Chlor, Fluor, Brom, Jod, -S(C₁-C₄-Alkyl) oder -SO₂(C₁-C₄-Alkyl) dar;
jedes R⁸ und R¹² ist unabhängig aus Wasserstoff und C₁-C₂-Alkyl ausgewählt;
jedes R⁴ und R⁶, das an ein Kohlenstoffatom gebunden ist, ist unabhängig aus Wasserstoff und C₁-C₆-Alkyl, Fluor, Chlor, Brom, Jod, Hydroxy, Hydroxy-(C₁-C₂-alkyl), Trifluormethyl, Cyano, Amino, Nitro, -O(C₁-C₄-Alkyl), -N(C₁-C₄-Alkyl)(C₁-C₂-alkyl), -CH₂SCH₃, -S(C₁-C₄-Alkyl), -CO(C₁-C₄-Alkyl), -C(=O)H oder -C(=O)O(C₁-C₄-Alkyl) ausgewählt, wobei jede der C₁-C₂-Alkyleinheiten in den vorangehenden Gruppen R⁴ und R⁶ gegebenenfalls eine Doppel- oder Dreifachbindung enthalten kann; und R⁶, wenn an ein Stickstoffatom gebunden, ist aus Wasserstoff und C₁-C₄-Alkyl ausgewählt;
R⁵ stellt substituiertes Phenyl, Pyridyl oder Pyrimidyl dar, worin jede der vorangehenden Gruppen R⁵ substituiert ist mit zwei oder drei Substituenten R¹³, wobei bis zu drei der Substituenten unabhängig ausgewählt sein können aus Chlor, C₁-C₄-Alkyl, -O(C₁-C₄-Alkyl), -(C₁-C₄-Alkylen)O(C₁-C₆alkyl), können unabhängig ausgewählt sein aus Fluor, Chlor, Brom, Jod, Formyl, Cyano, Trifluormethyl, OCF₃ und -(C₁-C₄-Alkylen)-OH, und worin das C₁-C₄-Alkyl gegebenenfalls eine Doppel- oder Dreifachbindung enthalten kann;
R⁷ stellt Wasserstoff, Methyl, Halogen (z.B. Chlor, Fluor, Jod oder Brom), Hydroxy, Methoxy, -C(=O)(C₁-C₂-Alkyl), -C(=O)O(C₁-C₂-Alkyl), Hydroxymethyl, Trifluormethyl oder Formyl dar;
R¹⁰ stellt Wasserstoff, Hydroxy, Methoxy oder Fluor dar; und
R¹¹ stellt Wasserstoff oder C₁-C₄-Alkyl dar;
mit der Maßgabe, dass es in dem Ring, der D, E, K und G von Formel I enthält, keine zwei zueinander benachbarte Doppelbindungen geben darf;
und das pharmazeutisch verträgliche Salz einer solchen Verbindung.

2. Verbindung nach Anspruch 1, worin A N, CH oder CH₃ darstellt.

3. Verbindung nach Anspruch 1, worin G Stickstoff darstellt.

4. Verbindung nach Anspruch 1, worin G Kohlenstoff darstellt und der Ring, der D, E, K und G enthält, ein Benzoring ist.

5. Verbindung nach Anspruch 1, worin G N darstellt; D NH, NCH₃ darstellt; und E⁻⁻⁻⁻K CH₂-CH₂, CH=CH, C(O)-CH₂ oder CH₂-C(O) darstellt.

6. Verbindung nach Anspruch 1, worin G N darstellt; D⁻⁻⁻⁻E⁻⁻⁻⁻K C(O)-O-CH₂, CH₂-O-CH₂, C(O)-CH₂-CH₂, C(O)-CH=CH, CH₂-CH₂-CH₂-, CH₂-CH₂-C(O), CH=CH-C(O), CH=CH-CH₂, CH=CH-NH, CH=CH-NCH₃ darstellt.

7. Verbindung nach Anspruch 1, worin R³ Methyl darstellt und jeder von R⁴, R⁶, R⁸, R⁹ und R¹² Wasserstoff darstellt.

8. Verbindung nach Anspruch 1, worin R⁵ di- oder tri-substituiertes Phenyl darstellt, worin die zwei oder drei Substituenten unabhängig ausgewählt sind aus C₁-C₄-Alkyl, -O- (C₁-C₄-Alkyl), -(C₁-C₄-Alkylen)-O-(C₁-C₄-alkyl), CF₃, -OCF₃, - CHO, -(C₁-C₄-Alkylen)-OH, Cyano, Chlor, Fluor, Brom und Jod, worin jede der vorangehenden (C₁-C₄)-Alkylgruppen gegebenenfalls eine Doppel- oder Dreifachbindung enthalten kann.

9. Verbindung nach Anspruch 1, worin R³ Methyl, Ethyl, Chlor oder Methoxy darstellt und jedes R⁴, R⁶, R⁸, R⁹ und R¹² unabhängig Wasserstoff oder Methyl darstellt.

10. Verbindung nach Anspruch 1, worin B -CHR¹R², -NCHR¹R² oder -OCHR¹R² darstellt, und die Gruppe CHR¹R² von B ein Cyclopentanring, ein Tetrahydrofuranring oder ein Tetrahydrothienylring ist.

11. Pharmazeutische Zusammensetzung für die Behandlung, Verhinderung oder Inhibierung von (a) einer Störung, deren Behandlung durch Entgegenwirken von CRF bewirkt oder erleichtert werden kann, einschließlich Störungen, die durch CRF induziert oder erleichtert werden, jedoch nicht begrenzt darauf oder (b) einer Störung, ausgewählt aus entzündlichen Störungen, wie rheumatische Arthritis und Osteoarthritis, Schmerz, Asthma, Psoriasis und Allergien, allgemeiner Angststörung; Panik; Phobien; obsessiver-kompulsiver Störung; post-traumatischer Stressstörung; Schlafstörungen, induziert durch Stress; Schmerzempfindung, wie Fibromyalgie; Stimmungsstörungen, wie Depression, einschließlich starker Depression, Einzelepisodendepression, wiederkehrender Depression, durch Kindsmissbrauch induzierter Depression, Stimmungsstörungen, verbunden mit premenstruellem Syndrom und Postpartumdepression; Dystemie; bipolaren Störungen; Cyclothymie; chronischem Ermüdungssyndrom; Stress-induziertem Kopfschmerz; Krebs; reizbarem Darmsyndrom, Crohn'scher Krankheit; spastischem Colon; post-operativem Ileus; Geschwür; Diarrhö; Stress-induziertem Fieber; Human-Immunmangel-Virus-(HIV)-Infektionen; neurodegenerativen Erkrankungen, wie Alzheimer-Krankheit, Parkinson-Krankheit und Huntington-Krankheit; gastrointestinalen Erkrankungen; Essstörungen, wie Anorexia und Bulimia nervosa; hämorrhagischem Stress; Chemikalienabhängigkeiten und Süchten (z.B. Abhängigkeiten von Alkohol, Kokain, Heroin, Benzodiazepinen oder anderen Arzneistoffen); Arzneistoff- und Alkoholentzugssymptomen; Stress-induzierten psychotischen Episoden; euthyroidem Krankheitssyndrom; Syndrom der inadäquaten ADH-Sekretion; Fettsucht; Infertilität; Schädeltraumen; Wirbelsäulentrauma; ischämischer neuronaler Schädigung (z.B. cerebraler Ischämie, wie cerebraler hippocampaler Ischämie); exzitotoxischer neuronaler Schädigung; Epilepsie; Schlaganfall; Immundysfunktionen, einschließlich Stress-induzierten Immundysfunktionen (z.B. Schweinestresssyndrom, Rindertransportfieber, paroxysmale Fibrillierung beim Pferd, und Dysfunktionen, induziert durch Beengung bei Hühnern, Scherstress beim Schaf oder durch Mensch-Tier-Wechselwirkung bedingter Stress bei Hunden); Muskelspasmen; Harninkontinenz; seniler Demenz vom Alzheimer-Typ; Multiinfarktdemenz; amyotropher lateraler Sklerose; Hypertension; Tachycardie; Herzstauungsinsuffizienz; Osteoporose; Frühgeburt; und Hypoglycämie bei einem Säuger, umfassend eine bei der Behandlung solcher Störung wirksame Menge einer Verbindung nach Anspruch 1 und einen pharmazeutisch verträglichen Träger.

12. Verwendung einer Verbindung nach Anspruch 1 für die Herstellung eines Arzneimittels zur Behandlung einer Störung nach Anspruch 11.

13. Verwendung einer Verbindung nach Anspruch 1 bei der Herstellung eines Arzneimittels zur Behandlung oder Verhinderung einer Störung oder eines Zustands, wobei die Behandlung oder Verhinderung davon durch Inhibieren des CRH-Bindungsproteins bei einem Säuger, einschließlich eines Menschen, bewirkt oder erleichtert werden kann, umfassend Verabreichen einer CRH-Bindungsprotein-inhibierenden Menge einer Verbindung nach Anspruch 1 an den Säuger.

14. Pharmazeutische Zusammensetzung zum Behandeln oder Verhindern einer Störung oder eines Zustands, wobei die Behandlung oder Verhinderung davon durch Inhibieren von CRH-Bindungsprotein bei einem Säuger, einschließlich eines Menschen, bewirkt oder erleichtert werden kann, umfassend eine CRH-Bindungsprotein-inhibierende Menge einer Verbindung nach Anspruch 1 und einen pharmazeutisch verträglichen Träger.

15. Verbindung nach Anspruch 1, worin G Kohlenstoff darstellt und der Ring, der D, E, K und G enthält, ein Benzoring ist.

16. Verbindung nach Anspruch 15, worin R³ Methyl darstellt.

17. Verbindung nach Anspruch 16, worin R⁵ an den ortho- und para-Stellungen di- oder tri-substituiertes Phenyl darstellt, wobei die zwei oder drei Substituenten unabhängig ausgewählt sind aus C₁-C₄-Alkyl, Cyclopropyl, -O-(C₁-C₄-Alkyl), -(C₁-C₄-Alkylen)-O-(C₁-C₄-alkyl), CF₃, OCF₃, CHO, - (C₁-C₄-Alkylen)-OH, Chlor, Fluor, Brom und Jod, worin jede der vorangehenden C₁-C₄-Alkylgruppen gegebenenfalls eine Doppel- oder Dreifachbindung enthalten kann.

18. Verbindung nach Anspruch 16, worin R⁵ an den ortho- oder para-Stellungen di- oder tri-substituiertes Pyridyl darstellt, wobei die zwei oder drei Substituenten unabhängig ausgewählt sind aus C₁-C₄-Alkyl, Cyclopropyl, -O-(C₁-C₄-Alkyl), -(C₁-C₄-Alkylen)-O-(C₁-C₄-alkyl), CF₃, OCF₃, CHO, - (C₁-C₄-Alkylen)-OH, Chlor, Fluor, Brom und Jod, worin jede der vorangehenden C₁-C₄-Alkylgruppen gegebenenfalls eine Doppel- oder Dreifachbindung enthalten kann.

19. Verbindung nach Anspruch 1, worin G N darstellt; D⁻⁻⁻E⁻⁻⁻K C(Cₙᵤₗₗ-C₁-Alkyl)-O-C(Cₙᵤₗₗ-C₁-alkyl)(Cₙᵤₗₗ-C₁-alkyl) darstellt.

20. Verbindung nach Anspruch 1, worin G N darstellt; D⁻⁻⁻⁻E⁻⁻⁻⁻K CH₂-O-CH₂ darstellt.

21. Verbindung nach Anspruch 20, worin R³ Methyl darstellt.

22. Verbindung nach Anspruch 21, worin R⁵ an den ortho- oder para-Stellungen di- oder tri-substituiertes Phenyl darstellt, wobei die zwei oder drei Substituenten unabhängig ausgewählt sind aus C₁-C₄-Alkyl, Cyclopropyl, -O-(C₁-C₄-Alkyl), -(C₁-C₄-Alkylen)-O-(C₁-C₄-alkyl), CF₃, OCF₃, CHO, -(C₁-C₄-Alkylen)-OH, Chlor, Fluor, Brom und Jod, worin jede der vorangehenden C₁-C₄-Alkylgruppen gegebenenfalls eine Doppeloder Dreifachbindung enthalten kann.

23. Verbindung nach Anspruch 21, worin R⁵ an den ortho- oder para-Stellungen di- oder tri-substituiertes Pyridyl darstellt, wobei die zwei oder drei Substituenten unabhängig ausgewählt sind aus C₁-C₄-Alkyl, Cyclopropyl, -O-(C₁-C₄-Alkyl), -(C₁-C₄-Alkylen)-O-(C₁-C₄-alkyl), CF₃, OCF₃, CHO, -(C₁-C₄-Alkylen)-OH, Chlor, Fluor, Brom und Jod, worin jede der vorangehenden C₁-C₄-Alkylgruppen gegebenenfalls eine Doppeloder Dreifachbindung enthalten kann.

24. Verbindung nach Anspruch 1, worin G N darstellt; D⁻⁻⁻⁻E⁻⁻⁻⁻K O-C(Cₙᵤₗₗ-C₁-Alkyl)(Cₙᵤₗₗ-C₁-alkyl)-C(Cₙᵤₗₗ-C₁-alkyl)(Cₙᵤₗₗ-C₁-alkyl), S-C(Cₙᵤₗₗ-C₁-Alkyl)(Cₙᵤₗₗ-C₁-alkyl)-C(Cₙᵤₗₗ-C₁-alkyl)(Cₙᵤₗₗ-C₁-alkyl) oder N(Cₙᵤₗₗ-C₁-Alkyl)-C(Cₙᵤₗₗ-C₁alkyl)(Cₙᵤₗₗ-C₁-alkyl)-C(Cₙᵤₗₗ-C₁-alkyl)(Cₙᵤₗₗ-C₁-alkyl) darstellt.

25. Verbindung nach Anspruch 1, worin G N darstellt; D⁻⁻⁻⁻E⁻⁻⁻⁻K O-CH₂-CH₂, O-CH=CH, S-CH₂-CH₂, S-CH=CH darstellt.

26. Verbindung nach Anspruch 25, worin R³ Methyl darstellt.

27. Verbindung nach Anspruch 26, worin R⁵ an den ortho- oder para-Stellungen di- oder tri-substituiertes Phenyl darstellt, wobei die zwei oder drei Substituenten unabhängig ausgewählt sind aus C₁-C₄-Alkyl, Cyclopropyl, -O-(C₁-C₄-Alkyl), -(C₁-C₄-Alkylen)-O-(C₁-C₄-alkyl), CF₃, OCF₃, CHO, - (C₁-C₄-Alkylen)-OH, Chlor, Fluor, Brom und Jod, worin jede der vorangehenden C₁-C₄-Alkylgruppen gegebenenfalls eine Doppel- oder Dreifachbindung enthalten kann.

28. Verbindung nach Anspruch 26, worin R⁵ an den ortho- oder para-Stellungen di-substituiertes Pyridyl darstellt, wobei die zwei oder drei Substituenten unabhängig ausgewählt sind aus C₁-C₄-Alkyl, Cyclopropyl, -O-(C₁-C₄-Alkyl), -(C₁-C₄-Alkylen)-O-(C₁-C₄-alkyl), CF₃, OCF₃, CHO, -(C₁-C₄-Alkylen)-OH, Chlor, Fluor, Brom und Jod, worin jede der vorangehenden C₁-C₄-Alkylgruppen gegebenenfalls eine Doppeloder Dreifachbindung enthalten kann.

29. Verbindung nach Anspruch 1, worin G N darstellt; D⁻⁻⁻⁻E⁻⁻⁻⁻K NH-CH₂-CH₂, NMe-CH₂-CH₂-N-R⁵, NH-CH=CH-N-R⁵ oder NCH₃-CH=CH-N-R⁵ darstellt.

30. Verbindung nach Anspruch 29, worin R³ Methyl darstellt.

31. Verbindung nach Anspruch 30, worin R⁵ an den ortho- oder para-Stellungen di- oder tri-substituiertes Phenyl darstellt, wobei die zwei oder drei Substituenten unabhängig ausgewählt sind aus C₁-C₄-Alkyl, Cyclopropyl, -O-(C₁-C₄-Alkyl), -(C₁-C₄-Alkylen)-O-(C₁-C₄-alkyl), CF₃, OCF₃, CHO, -(C₁-C₄-Alkylen)-OH, Chlor, Fluor, Brom und Jod, worin jede der vorangehenden C₁-C₄-Alkylgruppen gegebenenfalls eine Doppeloder Dreifachbindung enthalten kann.

32. Verbindung nach Anspruch 30, worin R⁵ an den ortho- oder para-Stellungen di-substituiertes Pyridyl darstellt, wobei die zwei oder drei Substituenten unabhängig ausgewählt sind aus C₁-C₄-Alkyl, Cyclopropyl, -O-(C₁-C₄-Alkyl), -(C₁-C₄-Alkylen)-O-(C₁-C₄-alkyl), CF₃, OCF₃, CHO, -(C₁-C₄-Alkylen)-OH, Chlor, Fluor, Brom und Jod, worin jede der vorangehenden C₁-C₄-Alkylgruppen gegebenenfalls eine Doppeloder Dreifachbindung enthalten kann.

33. Verbindung nach Anspruch 1, worin G N darstellt; D⁻⁻⁻E⁻⁻⁻K N=C (Cₙᵤₗₗ-C₁-Alkyl)-C(=O), N(Cₙᵤₗₗ-C₁-Alkyl)-C(=O)-C(Cₙᵤₗₗ-C₁-alkyl), C(=O)-N(Cₙᵤₗₗ-C₁-Alkyl)-C(=O), C(C_{I})=N-C(=O), C(Cₙᵤₗₗ-C₁-Alkyl)=N-C(=O), CH₂CH₂CH₂, CH₂-CH₂-C(=O), CH₂-N(Cₙᵤₗₗ-C₁-Alkyl)-C(=O) darstellt.

34. Verbindung nach Anspruch 33, worin R³ Methyl darstellt.

35. Verbindung nach Anspruch 34, worin R⁵ an den ortho- oder para-Stellungen di- oder tri-substituiertes Phenyl darstellt, wobei die zwei oder drei Substituenten unabhängig ausgewählt sind aus C₁-C₄-Alkyl, Cyclopropyl, -O-(C₁-C₄-Alkyl), -(C₁-C₄-Alkylen)-O-(C₁-C₄-alkyl), CF₃, OCF₃, CHO, -(C₁-C₄-Alkylen)-OH, Chlor, Fluor, Brom und Jod, worin jede der vorangehenden C₁-C₄-Alkylgruppen gegebenenfalls eine Doppeloder Dreifachbindung enthalten kann.

36. Verbindung nach Anspruch 34, worin R⁵ an den ortho- oder para-Stellungen di-substituiertes Pyridyl darstellt, wobei die zwei oder drei Substituenten unabhängig ausgewählt sind aus C₁-C₄-Alkyl, Cyclopropyl, -O-(C₁-C₄-Alkyl), -(C₁-C₄-Alkylen)-O-(C₁-C₄-alkyl), CF₃, OCF₃, CHO, -(C₁-C₄-Alkylen)-OH, Chlor, Fluor, Brom und Jod, worin jede der vorangehenden C₁-C₄-Alkylgruppen gegebenenfalls eine Doppeloder Dreifachbindung enthalten kann.

37. Verbindung nach Anspruch 1, worin die Verbindung ist:
4-(Butyl-ethyl-amino)-2,6-dimethyl-8-(2,4,6-trimethyl-phenyl)-5,8-dihydro-6H-pyrido[2,3-d]pyrimidin-7-on,
8-(1-Ethyl-propoxy)-6-methyl-4-(2,4,6-trimethyl-phenyl)-3,4-dihydro-1H-pyrido[2,3-b]pyrazin-2-on,
8-(1-Ethyl-propoxy)-6-methyl-4-(2,4,6-trimethyl-phenyl)-1,2,3,4-tetrahydro-pyrido[2,3-b]pyrazin,
8-(1-Ethyl-propoxy)-1,6-dimethyl-4-(2,4,6-trimethylphenyl)-3,4-dihydro-1H-pyrido[2,3-b]pyrazin-2-on,
5-(1-Ethyl-propoxy)-7-methyl-1-(2,4,6-trimethyl-phenyl)-1,4-dihydro-2H-3-oxa-1,8-diaza-naphthalin,
5-(1-Ethyl-propoxy)-7-methyl-1-(2,4,6-trimethyl-phenyl)-1,2-dihydro-3-oxa-1,8-diaza-naphthalin-4-on,
8-(1-Ethyl-propoxy)-1,6-dimethyl-4-(2,4,6-trimethylphenyl)-1,2,3,4-tetrahydro-pyrido[2,3-b]pyrazin,
(1-Ethyl-propyl)-[2-methyl-8-(2,4,6-trimethyl-phenyl)-chinolin-4-yl]-amin,
4-(1-Ethyl-propoxy)-2-methyl-8-(2,4,6-trimethyl-phenyl)-chinolin,
2-Methyl-4-(tetrahydro-furan-3-yloxy)-8-(2,4,6-trimethyl-phenyl)-chinolin,
5-(1-Ethyl-propoxy)-7-methyl-1-(2,4,6-trimethyl-phenyl)-3,4-dihydro-1H-[1,8]naphthyridin-2-on,
5-(1-Ethyl-propylamino)-7-methyl-1-(2,4,6-trimethylphenyl)-3,4-dihydro-1H-[1,8]naphthyridin-2-on,
5-(1-Ethyl-propoxy)-7-methyl-1-(2,4,6-trimethyl-phenyl)-3,4-dihydro-1H-pyrido-[2,3-d]pyrimidin-2-on,
4-(1-Ethyl-propoxy)-2,6-dimethyl-8-(2,4,6-trimethylphenyl)-8H-pteridin-7-on,
5-(1-Ethyl-propoxy)-7-methyl-1-(2,4,6-trimethyl-phenyl)-1,2,3,4-tetrahydro-[1,8]naphthyridin,
8-(1-Ethyl-propoxy)-2,6-dimethyl-4-(2,4,6-trimethylphenyl)-4H-pyrido-[2,3-b]pyrazin-3-on,
4-Chlor-5-(1-ethyl-propoxy)-7-methyl-1-(2,4,6-trimethyl-phenyl)-1H-pyrido-[2,3-d]pyrimidin-2-on,
5-(1-Ethyl-propoxy)-7-methyl-1-(2,4,6-trimethyl-phenyl)-1H-pyrido-[2,3-d]pyrimidin-2,4-dion,
1-(4-Brom-2,6-dimethyl-phenyl)-5-(1-ethyl-propoxy)-7-methyl-1,4-dihydro-2H-3-oxa-1,8-diaza-naphthalin,
1-(4-Chlor-2,6-dimethyl-phenyl)-5-(1-ethyl-propoxy)-7-methyl-1,4-dihydro-2H-3-oxa-1,8-diaza-naphthalin,
oder ein pharmazeutisch verträgliches Salz einer solchen Verbindung,

38. Verbindung der Formel 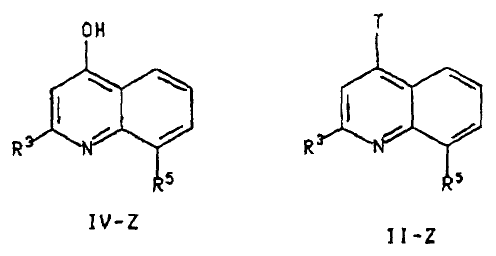 worin R³, R⁷ und R⁵ wie in Anspruch 1 definiert sind und T Cl, Br, J oder OTf darstellt.

## Revendications

1. Composé de formule 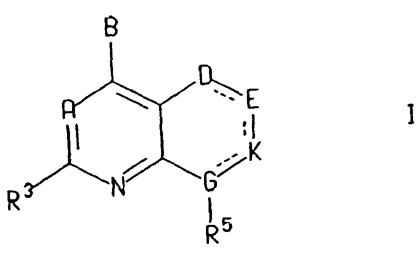 les lignes discontinues représentent des doubles liaisons facultatives ;
A représente un atome d'azote ou un groupe CR⁷ ;
B représente un groupe -NR¹R², NHCHR¹R², -OCHR¹R², CHR¹R² ou SCHR¹R² ;
G représente un atome d'azote ou un groupe CR⁴ et est lié par liaison simple à tous les atomes auxquels il est fixé, ou bien G représente un atome de carbone et est lié par une double liaison à K ;
K représente un atome d'azote ou un groupe CR⁶ lorsqu'il est lié par une double liaison à G ou E, ou bien K représente un groupe C=O, CR⁶R¹² ou NR⁸ lorsqu' il est lié par des liaisons simples aux deux atomes adjacents du noyau,
D et E représentent chacun, indépendamment, un groupe C=O, CR⁴R⁶ ou NR⁸ lors de la liaison par des liaisons simples aux deux atomes adjacents du noyau, ou un atome d'azote ou un groupe CR⁴ lors de la liaison par une double liaison à un atome adjacent du noyau ;
le noyau hexa- ou heptagonal qui contient D, E, K et G peut contenir une à trois doubles liaisons, zéro à deux hétéroatomes choisis entre des atomes d'oxygène, d'azote et de soufre, et zéro à deux groupes C=O ou C=S, les atomes de carbone de ces groupes faisant partie du noyau et les atomes d'oxygène et de soufre étant des substituants sur le noyau ;
R¹ représente un groupe alkyle en C₁ à C₆ facultativement substitué avec un substituant choisi entre des substituants hydroxy, fluoro, alkoxy en C₁ à C₄, CF₃ et cyclopropylfluoro,
où le groupe alkyle en C₁ à C₄ peut contenir facultativement une ou deux doubles ou triples liaisons ;
R² représente un groupe alkyle en C₁ à C₆ qui peut contenir facultativement une à trois doubles ou triples liaisons, (alkyle en C₁ ou C₂)-CO-(alkyle en C₁ ou C₂) ou benzyle, qui peut être facultativement substitué avec un substituant choisi entre des substituants chloro, fluoro, hydroxy, alkyle en C₁ ou C₂, CF₃, alkoxy en C₁ ou C₂ et cyclopropyl(alkyle en C₁ à C₄)-CO-(alkyle en C₁ à C₄) ;
-NR¹R² peut former un noyau choisi parmi des noyaux tri- à octogonaux saturés, dont les noyaux penta- à octogonaux peuvent contenir facultativement une ou deux doubles liaisons, et un ou deux des atomes de carbone du noyau de ces noyaux penta- à octogonaux peuvent être remplacés facultativement et indépendamment par un atome d'oxygène ou de soufre ou par un groupe NZ² dans lequel Z² représente un atome d'hydrogène, un groupe benzyle ou alkyle en C₁ à C₄ ;
R³ représente un atome d'hydrogène, un groupe alkyle en C₁ à C₄, -O(alkyle en C₁ à C₄), chloro, fluoro, bromo, iodo, -S(alkyle en C₁ à C₄) ou -SO₂(alkyle en C₁ à C₄) ;
chacun des groupes R⁸ et R¹² est choisi, indépendamment, entre un atome d'hydrogène et un groupe alkyle en C₁ ou C₂,
chacun des groupes R⁴ et R⁶ qui est fixé à un atome de carbone est choisi, indépendamment, entre un atome d'hydrogène et des groupes alkyle en C₁ à C₆, fluoro, chloro, bromo, iodo, hydroxy, hydroxyalkyle en C₁ ou C₂, trifluorométhyle, cyano, amino, nitro, -O(alkyle en C₁ à C₄), -N(alkyle en C₁ à C₄)(alkyle en C₁ ou C₂), -CH₂SCH₃, - S (alkyle en C₁ à C₄), -CO(alkyle en C₁ à C₄), -C(=O)H ou -C(=O)O(alkyle en C₁ à C₄), dans lesquels chacun des groupements alkyle en C₁ ou C₂ dans les groupes R⁴ et R⁶ précités peut contenir facultativement une double ou triple liaison ; et R⁶, lorsqu'il est fixé à un atome d'azote, est choisi entre un atome d'hydrogène et un groupe alkyle en C₁ à C₄ ;
R⁵ représente un groupe phényle, pyridyle ou pyrimidyle substitué, chacun des groupes R⁵ précités étant substitué avec deux ou trois substituants R¹³, jusqu'à trois desdits substituants pouvant être choisis, indépendamment, entre des substituants chloro, alkyle en C₁ à C₄, -O(alkyle en C₁ à C₄), -(alkylène en C₁ à C₄)O(alkyle en C₁ à C₆),et un desdits substituants pouvant être choisi, indépendamment, entre des substituants fluoro, chloro, bromo, iodo, formyle, cyano, trifluorométhyle, OCF₃ et -(alkylène en C₁ à C₄)-OH, et le groupe alkyle en C₁ à C₄ peut contenir facultativement une double ou triple liaison ;
R⁷ représente un atome d'hydrogène, un groupe méthyle, halogéno (par exemple chloro, fluoro, iodo ou bromo), hydroxy, méthoxy, -C(=O)(alkyle en C₁ ou C₂), -C(=O)O(alkyle en C₁ ou C₂), hydroxyméthyle, trifluorométhyle ou formyle ;
R¹⁰ représente un atome d'hydrogène, un groupe hydroxy, méthoxy ou fluoro ; et
R¹¹ représente un atome d'hydrogène ou un groupe alkyle en C₁ à C₄ ;
sous réserve que, dans le noyau contenant D, E, K et G de la formule 1, il ne puisse exister deux doubles liaisons adjacentes l'une à l'autre ;
et le sel pharmaceutiquement acceptable d'un tel composé.

2. Composé suivant la revendication 1, dans lequel A représente un atome de N ou un groupe CH ou CH₃.

3. Composé suivant la revendication 1, dans lequel G représente un atome d'azote.

4. Composé suivant la revendication 1, dans lequel G représente un atome de carbone et le noyau contenant D, E, K et G est un noyau benzo.

5. Composé suivant la revendication 1, dans lequel G représente un atome de N ; D représente un groupe NH, NCH₃ ; et E---K représente un groupe CH₂-CH₂, CH=CH, C(O)-CH₂ ou CH₂-C(O).

6. Composé suivant la revendication 1, dans lequel G représente un atome de N ; D---E---K représente un groupe C(O)-O-CH₂, CH₂-O-CH₂, C(O)-CH₂-CH₂, C(O)-CH=CH, CH₂-CH₂-CH₂, CH₂-CH₂-C(O), CH=CH-C(O), CH=CH-CH₂, CH=CH-NH, CH=CH-NCH₃.

7. Composé suivant la revendication 1, dans lequel R³ représente un groupe méthyle et chacun des groupes R⁴, R⁶, R⁸, R⁹ et R¹² représente un atome d'hydrogène.

8. Composé suivant la revendication 1, dans lequel R⁵ représente un groupe phényle di- ou tri-substitué dans lequel les deux ou trois substituants sont choisis indépendamment entre des substituants alkyle en C₁ à C₄, -O-(alkyle en C₁ à C₄), -(alkylène en C₁ à C₄)-O-(alkyle en C₁ à C₄), CF₃, -OCF₃, -CHO, -(alkylène en C₁ à C₄)-OH, cyano, chloro, fluoro, bromo ou iodo, chacun des groupes alkyle en C₁ à C₄ précités pouvant contenir facultativement une double ou triple liaison.

9. Composé suivant la revendication 1, dans lequel R³ représente un groupe méthyle, éthyle, chloro ou méthoxy et chacun des groupes R⁴, R⁶, R⁸, R⁹ et R¹² représentent, indépendamment, un atome d'hydrogène ou un groupe méthyle.

10. Composé suivant la revendication 1, dans lequel B représente un groupe -CHR¹R², -NCHR¹R² ou -OCHR¹R², et le groupe CHR¹R² de B est un noyau cyclopentane, un noyau tétrahydrofuranne ou un noyau tétrahydrothiényle.

11. Composition pharmaceutique pour le traitement, la prévention ou l'inhibition (a) d'un trouble dont le traitement peut être effectué ou facilité par antagonisation du CRF, comprenant, mais à titre non limitatif, des troubles induits ou facilités par le CRF, ou (b) d'un trouble choisi entre des troubles inflammatoires tels que la polyarthrite rhumatoïde et l'ostéoarthrite, la douleur, l'asthme, le psoriasis et des allergies ; l'anxiété généralisée ; la panique; des phobies ; le trouble obsessionnel compulsif ; le syndrome de stress post-traumatique ; des troubles du sommeil induits par un stress ; une perception douloureuse telle que la fibromyalgie ; des troubles de l'humeur tels que la dépression, comprenant la dépression majeure, la dépression à épisode unique, la dépression récidivante, la dépression induite par des sévices sur enfants, des troubles de l'humeur associés au syndrome prémenstruel, et la dépression du postpartum ; la dysthimie ; des troubles bipolaires; la cyclothymie ; le syndrome de fatigue chronique; les céphalées induites par un stress ; le cancer; le syndrome du côlon irritable; la maladie de Crohn; le côlon spastique; une occlusion intestinale post-opératoire; un ulcère; la diarrhée; la fièvre induite par un stress ; des infections par le virus d'immunodéficience humaine (VIH) ; des maladies neuro-dégénératives telles que la maladie d'Alzheimer, la maladie de Parkinson et la maladie de Huntington ; des maladies gastro-intestinales ; des troubles de l'alimentation tels que l'anorexie mentale et la boulimie mentale ; un stress hémorragique ; des dépendances et accoutumances chimiques (par exemple des dépendances à l'alcool, la cocaïne, l'héroïne, les benzodiazépines ou d'autres médicaments) ; des symptômes de sevrage de médicaments et de l'alcool ; des épisodes psychotiques induits par un stress ; le syndrome de maladie euthyroïdienne ; le syndrome d'hormone anti-diarrhéique inappropriée (ADH); l'obésité ; la stérilité ; des traumatismes crâniens; un traumatisme de la moelle épinière ; une altération neuronale ischémique (par exemple une ischémie cérébrale telle que l'ischémie hippocampique cérébrale) ; une altération neuronale excitotoxique; l'épilepsie ; un ictus ; des dysfonctionnements immunitaires induits par un stress (par exemple le syndrome de stress porcin, la fièvre des transports des bovins, la fibrillation paroxystique équine et des dysfonctionnements induits par le confinement chez les poulets, le stress des embardées lors des transports des moutons ou le stress dû à une interaction homme-animal chez le chien) ; les spasmes musculaires ; l'incontinence urinaire ; la démence sénile de type Alzheimer ; la démence artériopathique; la sclérose latérale amyotrophique ; l'hypertension; la tachycardie ; l'insuffisance cardiaque congestive ; l'ostéoporose ; la naissance prématurée ; l'hypoglycémie chez un mammifère, comprenant une quantité d'un composé suivant la revendication 1 qui est efficace dans le traitement d'un tel trouble, et un support pharmaceutiquement acceptable.

12. Utilisation d'un composé suivant la revendication 1 pour la préparation d'un médicament destiné au traitement d'un trouble suivant la revendication 11.

13. Utilisation d'un composé suivant la revendication 1 dans la préparation d'un médicament destiné au traitement ou à la prévention d'un trouble ou d'une affection, dont le traitement ou la prévention peut être effectué ou facilité en inhibant la protéine de liaison de CRH chez un mammifère, y compris l'homme, comprenant l'administration audit mammifère d'une quantité, inhibant la protéine de liaison de CRH, d'un composé suivant la revendication 1.

14. Composition pharmaceutique pour le traitement ou la prévention d'un trouble ou d'une affection, dont le traitement ou la prévention peut être effectué ou facilité en inhibant la protéine de liaison de CRH chez un mammifère, y compris l'homme, comprenant une quantité inhibitrice de la protéine de liaison de CRH d'un composé suivant la revendication 1 et un support pharmaceutiquement acceptable.

15. Composé suivant la revendication 1, dans lequel G représente un atome de carbone et le noyau contenant D, E, K et G est un noyau benzo.

16. Composé suivant la revendication 15, dans lequel R³ représente un groupe méthyle.

17. Composé suivant la revendication 16, dans lequel R⁵ représente un groupe phényle di- ou tri-substitué en positions ortho ou para, dans lequel les deux ou trois substituants sont choisis indépendamment entre des substituants alkyle en C₁ à C₄, cyclopropyle, -O-(alkyle en C₁ à C₄), -(alkylène en C₁ à C₄)-O-(alkyle en C₁ à C₄), CF₃, OCF₃, CHO, -(alkylène en C₁ à C₄)-OH, chloro, fluoro, bromo et iodo, dans lesquels chacun des groupes alkyle en C₁ à C₄ précités peut contenir facultativement une double ou triple liaison.

18. Composé suivant la revendication 16, dans lequel R⁵ représente un groupe pyridyle di- ou tri-substitué en positions ortho ou para, dans lequel les deux ou trois substituants sont choisis indépendamment entre des substituants alkyle en C₁ à C₄, cyclopropyle, -O-(alkyle en C₁ à C₄), -(alkylène en C₁ à C₄)-O-(alkyle en C₁ à C₄), CF₃, OCF₃, CHO, -(alkylène en C₁ à C₄)-OH, chloro, fluoro, bromo et iodo, dans lesquels chacun des groupes alkyle en C₁ à C₄ précités peut contenir facultativement une double ou triple liaison.

19. Composé suivant la revendication 1, dans lequel G représente un atome de N ; D---E---K représente un groupe C(alkyle en C_{zéro} ou C₁)-O-C(alkyle en C_{zéro} ou C₁)(alkyle en C_{zéro} ou C₁).

20. Composé suivant la revendication 1, dans lequel G représente un atome de N ; D---E---K représente un groupe CH₂-O-CH₂.

21. Composé suivant la revendication 20, dans lequel R³ représente un groupe méthyle.

22. Composé suivant la revendication 21, dans lequel R⁵ représente un groupe phényle di- ou tri-substitué en positions ortho ou para, dans lequel les deux ou trois substituants sont choisis indépendamment entre des substituants alkyle en C₁ à C₄, cyclopropyle, -O-(alkyle en C₁ à C₄), -(alkylène en C₁ à C₄)-O-(alkyle en C₁ à C₄), CF₃, OCF₃, CHO, -(alkylène en C₁ à C₄)-OH, chloro, fluoro, bromo et iodo, dans lesquels chacun des groupes alkyle en C₁ à C₄ précités peut contenir facultativement une double ou triple liaison.

23. Composé suivant la revendication 21, dans lequel R⁵ représente un groupe pyridyle di- ou tri-substitué en positions ortho ou para, dans lequel les deux ou trois substituants sont choisis indépendamment entre des substituants alkyle en C₁ à C₄, cyclopropyle, -O-(alkyle en C₁ à C₄), -(alkylène en C₁ à C₄)-O-(alkyle en C₁ à C₄), CF₃, OCF₃, CHO, -(alkylène en C₁ à C₄)-OH, chloro, fluoro, bromo et iodo, dans lesquels chacun des groupes alkyle en C₁ à C₄ précités peut contenir facultativement une double ou triple liaison.

24. Composé suivant la revendication 1, dans lequel G représente un atome de N ; D---E---K représente un groupe O-C(alkyle en C_{zéro} ou C₁)(alkyle en C_{zéro} ou C₁)-C(alkyle en C_{zéro} ou C₁)(alkyle en C_{zéro} ou C₁), S-C (alkyle en C_{zéro} ou C₁)(alkyle en C_{zéro} ou C₁)-C(alkyle en C_{zéro} ou C₁)(alkyle en C_{zéro} ou C₁) ou N(alkyle en C_{zéro} ou C₁)-C(alkyle en C_{zéro} ou C₁)-C(alkyle en C_{zéro} ou C₁)(alkyle en C_{zéro} ou C₁).

25. Composé suivant la revendication 1, dans lequel G représente un atome de N ; D---E---K représentent un groupe O-CH₂-CH₂, O-CH=CH, S-CH₂-CH₂ ou -S-CH=CH.

26. Composé suivant la revendication 25, dans lequel R³ représente un groupe méthyle.

27. Composé suivant la revendication 26, dans lequel R⁵ représente un groupe phényle di- ou tri-substitué en positions ortho ou para, dans lequel les deux ou trois substituants sont choisis indépendamment entre des substituants alkyle en C₁ à C₄, cyclopropyle, -O-(alkyle en C₁ à C₄), -(alkylène en C₁ à C₄)-O-(alkyle en C₁ à C₄), CF₃, OCF₃, CHO, -(alkylène en C₁ à C₄)-OH, chloro, fluoro, bromo et iodo, dans lesquels chacun des groupes alkyle en C₁ à C₄ précités peut contenir facultativement une double ou triple liaison.

28. Composé suivant la revendication 26, dans lequel R⁵ représente un groupe pyridyle di-substitué en positions ortho ou para, dans lequel les deux ou trois substituants sont choisis indépendamment entre des substituants alkyle en C₁ à C₄, cyclopropyle, -O-(alkyle en C₁ à C₄), -(alkylène en C₁ à C₄)-O-(alkyle en C₁ à C₄), CF₃, OCF₃, CHO, -(alkylène en C₁ à C₄)-OH, chloro, fluoro, bromo et iodo, dans lesquels chacun des groupes alkyle en C₁ à C₄ précités peut contenir facultativement une double ou triple liaison.

29. Composé suivant la revendication 1, dans lequel G représente un atome de N ; D---E---K représente un groupe NH-CH₂-CH₂, NMe-CH₂-CH₂-N-R⁵, NH-CH=CH-N-R⁵ ou NCH₃-CH=CH-N-R⁵.

30. Composé suivant la revendication 29, dans lequel R³ représente un groupe méthyle.

31. Composé suivant la revendication 30, dans lequel R⁵ représente un groupe phényle di- ou tri-substitué en positions ortho ou para, dans lequel les deux ou trois substituants sont choisis indépendamment entre des substituants alkyle en C₁ à C₄, cyclopropyle, -O-(alkyle en C₁ à C₄), -(alkylène en C₁ à C₄)-O-(alkyle en C₁ à C₄), CF₃, OCF₃, CHO, -(alkylène en C₁ à C₄)-OH, chloro, fluoro, bromo et iodo, dans lesquels chacun des groupes alkyle en C₁ à C₄ précités peut contenir facultativement une double ou triple liaison.

32. Composé suivant la revendication 30, dans lequel R⁵ représente un groupe pyridyle di-substitué en positions ortho ou para, dans lequel les deux ou trois substituants sont choisis indépendamment entre des substituants alkyle en C₁ à C₄, cyclopropyle, -O-(alkyle en C₁ à C₄), -(alkylène en C₁ à C₄)-O-(alkyle en C₁ à C₄), CF₃, OCF₃, CHO, -(alkylène en C₁ à C₄)-OH, chloro, fluoro, bromo et iodo, dans lesquels chacun des groupes alkyle en C₁ à C₄ précités peut contenir facultativement une double ou triple liaison.

33. Composé suivant la revendication 1, dans lequel G représente un atome de N ; D---E---K représente un groupe N=C(alkyle en C_{zéro} ou C₁)-C(=O), N(alkyle en C_{zéro} ou C₁)-C(=O)-C(alkyle en C_{zéro} ou C₁), C(=O)-N(alkyle en C_{zéro} ou C₁)-C(=O), C(C₁)=N-C(=O), C(alkyle en C_{zéro} ou C₁)=N-C(=O), CH₂-CH₂-CH₂, CH₂-CH₂-C(=O) ou CH₂-N(alkyle en C_{zéro} ou C₁)-C(=O).

34. Composé suivant la revendication 33, dans lequel R³ représente un groupe méthyle.

35. Composé suivant la revendication 34, dans lequel R⁵ représente un groupe phényle di- ou tri-substitué en positions ortho ou para, dans lequel les deux ou trois substituants sont choisis indépendamment entre des substituants alkyle en C₁ à C₄, cyclopropyle, -O-(alkyle en C₁ à C₄), -(alkylène en C₁ à C₄)-O-(alkyle en C₁ à C₄), CF₃, OCF₃, CHO, -(alkylène en C₁ à C₄)-OH, chloro, fluoro, bromo et iodo, dans lesquels chacun des groupes alkyle en C₁ à C₄ précités peut contenir facultativement une double ou triple liaison.

36. Composé suivant la revendication 34, dans lequel R⁵ représente un groupe pyridyle di-substitué en positions ortho ou para, dans lequel les deux ou trois substituants sont choisis indépendamment entre des substituants alkyle en C₁ à C₄, cyclopropyle, -O-(alkyle en C₁ à C₄), -(alkylène en C₁ à C₄)-O-(alkyle en C₁ à C₄), CF₃, OCF₃, CHO, -(alkylène en C₁ à C₄)-OH, chloro, fluoro, bromo et iodo, dans lesquels chacun des groupes alkyle en C₁ à C₄ précités peut contenir facultativement une double ou triple liaison.

37. Composé suivant la revendication 1, ledit composé étant :
la 4-(butyl-éthylamino)-2,6-diméthyl-8-(2,4,6-triméthylphényl)-5,8-dihydro-6H-pyrido[2,3-d]pyrimidine-7-one ;
la 8-(1-éthylpropoxy)-6-méthyl-4-(2,4,6-triméthylphényl)-3,4-dihydro-1H-pyrido[2,3-d]pyrazine-2-one ;
la 8-(1-éthylpropoxy)-6-méthyl-4-(2,4,6-triméthylphényl)-1,2,3,4-tétrahydro-pyrido[2,3-b]pyrazine ;
la 8-(1-éthylpropoxy)-1,6-diméthyl-4-(2,4,6-triméthylphényl)-3,4-dihydro-1H-pyrido[2,3-b]pyrazine-2-one ;
le 5-(1-éthylpropoxy)-7-méthyl-1-(2,4,6-triméthylphényl)-1,4-dihydro-2H-3-oxa-1,8-diaza-naphtalène ;
la 5-(1-éthylpropoxy)-7-méthyl-1-(2,4,6-triméthylphényl)-1,2-dihydro-3-oxa-1,8-diaza-naphtalène-4-one ;
le 8-(1-éthylpropoxy)-1,6-diméthyl-4-(2,4,6-triméthylphényl)-1,2,3,4-tétrahydro-pyrido[2,3-b]pyrazine ;
la (1-éthylpropyl)-[2-méthyl-8-(2,4,6-triméthylphényl)-quinoléine-4-yl]-amine ;
la 4-(1-éthylpropoxy) -2-méthyl-8- (2, 4, 6-triméthylphényl)-quinoléine ;
la 2-méthyl-4-(tétrahydrofuranne-3-ytoxy)-8-(2,4,6-triméthylphényl)-quinoléine ;
la 5-(1-éthylpropoxy)-7-méthyl-1-(2,4,6-triméthylphényl)-3,4-dihydro-1H-[1,8]naphtyridine-2-one ;
la 5-(1-éthyl-propylamino)-7-méthyl-1-(2,4,6-triméthylphényl)-3,4-dihydro-1H-[1,8]naphtyridine-2-one ;
la 5-(1-éthylpropoxy)-7-méthyl-1-(2,4,6-triméthylphényl)-3,4-dihydro-1H-pyrido[2,3-d]pyrimidine-2-one ;
la 4-(1-éthylpropoxy)-2,6-diméthyl-8-(2,4,6-triméthylphényl)-8H-ptéridine-7-one ;
la 5-(1-éthylpropoxy)-7-méthyl-1-(2,4,6-triméthylphényl)-1,2,3,4-tétrahydro-[1,8]naphtyridine ;
la 8-(1-éthylpropoxy)-2,6-diméthyl-4-(2,4,6-triméthylphényl)-4H-pyrido-[2,3-b]pyrazine-3-one ;
la 4-chloro-5-(1-éthylpropoxy)-7-méthyl-1-(2,4,6-triméthylphényl)-1H-pyrido--[2,3-d]pyrimidine-2-one ;
la 5-(1-éthylpropoxy)-7-méthyl-1-(2,4,6-triméthylphényl)-1H-pyrido-[2,3-d]pyrimidine-2,4-dione ;
le 1-(4-bromo-2,6-diméthylphényl)-5-(1-éthylpropoxy)-7-méthyl-1,4-dihydro-2H-3-oxa-1,8-diaza-naphtalène ;
le 1-(4-chloro-2,6-diméthylphényl)-5-(1-éthylpropoxy)-7-méthyl-1,4-dihydro-2H-3-oxa-1,8-diaza-naphtalène ;
ou un sel pharmaceutiquement acceptable d'un tel composé.

38. Composé de formule 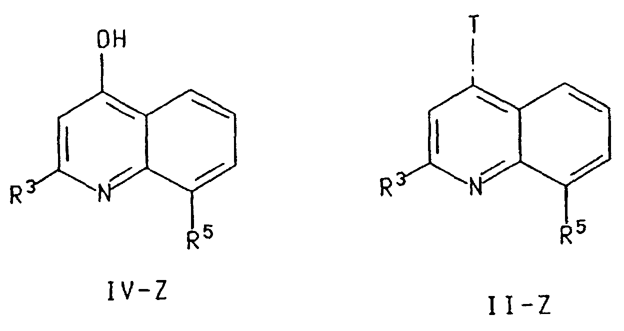 dans laquelle R³, R⁷ et R⁵ répondent aux définitions suivant la revendication 1 et T représente un groupe Cl, Br, I ou OTf.
